# EUROPEAN PATENT SPECIFICATION

(11) **EP 3 783 027 B1**
(45) Date of publication and mention of the grant of the patent: **03.12.2025**
(21) Application number: 20177260.5
(22) Date of filing: 21.10.2015
(51) Int. Cl.: C07K 16/28, A61P 17/02, A61P 29/00, A61P 31/00, A61P 35/00, A61P 35/04, A61P 37/04, A61P 37/06, A61P 43/00, A61K 39/00

(54) **METHOD AND COMPOSITIONS FOR INDUCING DIFFERENTIATION OF MYELOID DERIVED SUPPRESSOR CELL TO TREAT CANCER**
VERFAHREN UND ZUSAMMENSETZUNGEN ZUR INDUZIERUNG DER DIFFERENZIERUNG VON AUS MYELOIDEN GEWONNENEN SUPPRESSORZELLEN ZUR BEHANDLUNG VON KREBS
PROCÉDÉ ET COMPOSITIONS POUR INDUIRE LA DIFFÉRENCIATION DE CELLULE SUPPRESSIVES DÉRIVÉES DE MYÉLOÏDE POUR TRAITER LE CANCER

(30) Priority: 24.10.2014 EP 14190370
(43) Date of publication of application: 24.02.2021
(62) Divisional of application: 15794641.9
(73) Proprietor: OSE Immunotherapeutics, 44200 Nantes (FR)
(72) Inventor: POIRIER, Nicolas, 44119 Treillieres (FR); VANHOVE, Bernard, 44400 Reze (FR)
(74) Representative: Ernest Gutmann - Yves Plasseraud S.A.S.

(56) References cited:
- WO-A1-2013/056352
- WO-A2-2012/149416
- CHAO M P ET AL: "Response: Mechanisms of targeting CD47-SIRP[alpha] in hematologic malignancies", BLOOD, AMERICAN SOCIETY OF HEMATOLOGY, US, vol. 119, no. 18, 3 May 2012 (2012-05-03), pages 4334 - 4335, XP009160297, ISSN: 0006-4971, DOI: 10.1182/BLOOD-2012-02-411249
- JORDI C OCHANDO ET AL: "Myeloid-derived suppressor cells in transplantation and cancer", IMMUNOLOGIC RESEARCH, HUMANA PRESS INC, NEW YORK, vol. 54, no. 1 - 3, 26 April 2012 (2012-04-26), pages 275 - 285, XP035126289, ISSN: 1559-0755, DOI: 10.1007/S12026-012-8335-1
- ZHAO XI WEN ET AL: "CD47-signal regulatory protein-[alpha] (SIRP[alpha]) interactions form a barrier for antibody-mediated tumor cell destruction", PROCEEDINGS OF THE NATIONAL ACADEMY OF SCIENCES (PNAS), vol. 108, no. 45, 31 October 2011 (2011-10-31), pages 18342 - 18347, XP093286277, ISSN: 0027-8424, DOI: 10.1073/pnas.1106550108

## Description

The present invention pertains to the field of immunotherapy. More specifically, the present invention provides a compound selected from the group consisting of an antibody and an antigen-binding fragment thereof, wherein the compound specifically binds to the signal regulatory protein alpha (SIRPa), and blocks the interaction between SIRPa and CD47, for use in the treatment of a patient with a solid cancer in a patient, the solid cancer being selected from the group consisting of carcinoma, adenocarcinoma, breast cancer, prostate cancer, ovarian cancer, lung cancer, pancreatic cancer, colon cancer, sarcoma, lymphoma and blastoma..

Myeloid-derived suppressor cells (MDSC) are a group of heterogeneous cells of immature hematopoietic myeloid-cell progenitors, exerting immunosuppressive functions and therefore negatively regulating immune responses (Gabrilovich and Nagaraj, 2009; Talmadge and Gabrilovich, 2013). MDSC were found to be accumulated in many pathological conditions and to exploit a plethora of redundant mechanisms to influence both innate and adaptive immune responses (Dilek et al., 2010; Gabrilovich et al., 2012). MDSC are potent inhibitors of T and B lymphocytes activation, proliferation and responses, in particular by nutrients (e.g. L-arginine and L-cysteine) depletion mechanisms, the production of reactive oxygen species (ROS) and reactive nitrogen species (RNS), perturbation of T-lymphocyte trafficking (e.g. L-selectin expression decrease and aberrant chemokine release), induction of apoptosis (via Galectin 9) and by deviating T-lymphocytes differentiation towards Th-17 responses through IL-1 β production (Bruchard et al., Nat. Med. 2013). MDSC have also the extraordinary capacity to expand antigen-specific natural regulatory T cells (nTreg), to promote conversion of naive T cells into induced Treg (iTreg) cells and to promote Treg infiltration at inflamed, infected or tumor sites. MDSC were also described to decrease the number and inhibit function of NK cells, in particular by membrane bound TGFβ. Furthermore, in analogy with the immune deviation they induce in T cells responses, MDSC skew macrophages towards an M2 phenotype (non-inflammatory macrophages) by inhibiting macrophages production of IL-12. Similarly, MDSC impair dendritic cell (DC) function by producing IL-10, which also inhibits IL-12 production by DC and reduces DC capacity to activate T cells. Finally, MDSC act on non-hematopoietic cell and have been in particular widely recognized to facilitate tumor angiogenesis, tumor spread, tumor-cell invasion and metastasis (Keskinov and Shurin, 2014; Ye et al., 2010).

In physiological condition, hematopoietic stem cells differentiate into common lymphoid progenitor (CLP) or common myeloid progenitor (CMP) cells. These progenitors then generate the two distinct types of cells of our immune system: CLP differentiate into lymphocytes or natural killer (NK) cells, while CMP differentiate into immature myeloid cells (IMCs). Normally, IMCs migrate to different peripheral organs, where they differentiate exclusively into dendritic cells and macrophages or polymorphonuclear cells (also named granulocytes). However, several factors (e.g. GM-CSF, M-CSF, IL-6, IL-1β, IL-13, S100A8/A9 *etc*.) produced in many pathological conditions, promote the accumulation of IMCs, prevent their differentiation and induce their activation. These cells exhibit immunosuppressive functions after activation and were named MDSC in 2007 (Gabrilovich et al., 2007). So far, three main MDSC populations have been phenotypically and functionally characterized: pro-myelocytic or monocytic MDSC (M-MDSC or Mo-MDSC) and polymorphonuclear (also called granulocytic) MDSC (PMN-MDSC or G-MDSC). Mo-MDSC, characterized by the CD11b⁺ Ly6C⁺ Ly6G⁻ phenotype in mice and CD11b⁺ CD33⁺ HLA-DR^{-/low} CD14⁺ phenotype in humans, are the most potent immunosuppressive MDSC population, function at least by expressing nitrite oxide synthase (iNOS) and arginase (ARG1) enzymes, and are abundant in the tumor microenvironment. Pro-myelocytic MDSC resemble Mo-MDSC but do not express the CD14 marker, suggesting a more immature state as compared to Mo-MDSC (Diaz-Montero et al., 2014). PMN-MDSC, characterized by the CD11b⁺ Ly6C^{low} Ly6G⁺ phenotype in mice and CD11b⁺ CD33⁺ HLA-DR⁻ CD15⁺ phenotype in humans, are more predominant in the periphery and lymphoid organs, and function mainly by producing reactive oxygen species (ROS) (Solito et al., 2014).While MDSC are precursors of macrophages, dendritic cells or granulocytes blocked in their differentiation in some pathological conditions, they still have the potential to pursue their "normal" differentiation road. In fact, culture of Mo-MDSC in the absence of inflammatory or tumor-derived soluble factors, as well as transfer into naive healthy host, differentiate these cells into macrophages or dendritic cells. Furthermore, hypoxic conditions (such as the tumor microenvironment), could drive their differentiation into immunosuppressive M2-like tumor-associated macrophages (TAM). Similarly, after 24 hours of culture in the absence of inflammatory or tumor-derived soluble factors, PMN-MDSC phenotypically and functionally resemble mature granulocytes (Gabrilovich et al., 2012). Furthermore, PMN-MDSC, which have a relatively shorter lifespan and lower proliferation ability than Mo-MDSC, could be replenished from Mo-MDSC in pathological settings (Youn et al., 2013).

MDSC are now considered as key cells expanding in pathological situations and preventing adequate immune responses and are associated with significant morbidities and co-morbidities in a large number of diseases. First, a growing number of studies demonstrated that MDSC levels correlate with cancer stages severity, metastasis and are independently prognostic of overall survival in patients suffering from diverse cancers: breast, colon, melanoma, lung, liver, gastric, renal, pancreas, bladder, prostate, ovarian, esophageal, sarcoma, glioblastoma, head and neck cancers, as well as lymphoma, leukemia and myeloma hematological cancers (Diaz-Montero et al., 2009; Gabitass et al., 2011; Huang et al., 2013; Idorn et al., 2014; Kalathil et al., 2013; Khaled et al., 2013; Kitano et al., 2014; Shen et al., 2014; Solito et al., 2014; Sun et al., 2012; Weide et al., 2014; Zhang et al., 2013). MDSC have now a clear prognostic importance in multiple cancers, and emerging data support the utility of circulating MDSCs as a predictive biomarker for cancer immunotherapy, and even as an early leading marker for predicting clinical response to systemic chemotherapy in patients with advanced solid tumors (Kitano et al., 2014; Weide et al., 2014). Based on these clinical studies and numbers of preclinical reports, targeting MDSC either in combination with cancer immunotherapy, chemotherapy or independently as part of an approach to inhibit the metastatic process appears to be a very clinically promising strategy (Diaz-Montero et al., 2014).

MDSC accumulation has also been documented in non-cancer settings. The evasion of host immunity employed by pathogens to establish persistence and chronic infection strikingly parallels mechanisms of tumor escape. Hence, MDSC have been reported in a variety of infectious diseases, including bacterial (e.g. *Pseudomonas aeruginosa, Listeria monocytogenes, Mycobacterium tuberculosis, Staphylococcus aureus,* bacterial pneumonia), parasitic (e.g. *Leishmania),* fungal (e.g. *Candida*)*,* and viral (Hepatitis B and C, HIV, *Influenza,* herpes) infections and have been associated with persistence of chronic infection (Cai et al., 2013; Goh et al., 2013; Van Ginderachter et al., 2010; Vollbrecht et al., 2012). In fact, proinflammatory cytokines produced during the acute phase of infection and pathogens-derived particles activator of Toll like receptor (TLR) were described to induce and expand MDSC which will dampen the appropriate immune responses and are co-responsible of the chronic infection. Furthermore, MDSC generated during viral infections are particularly interesting, because many viruses are also oncogenic and, together, will induce locally dysfunctional inflammatory environment similar to that of tumors.

Similarly to infectious disease or cancer, MDSC also play a significant deleterious role in vaccination, both against pathogens or tumor antigen. For example, on the one hand, antigens as well as adjuvants used in HIV-vaccine composition activate and expand MDSC (Garg and Spector, 2014; Sui et al., 2014). On the other hand, protective immunity after *Salmonella* vaccine in patients directly correlates with reduced expansion of MDSC (Heithoff et al., 2008) and depletion of MDSC significantly augments antitumor immunity after therapeutic vaccination (Srivastava et al., 2012a, 2012b). High levels of MDSC have been also documented in traumatized, injured or burnt patients and high levels of these immature myeloid cells were significantly associated with high risk of morbidities, in particular caused by sepsis (Cheron et al., 2010; Cuenca et al., 2011; Janols et al., 2014; Makarenkova et al., 2006; Taylor et al., 2000; Venet et al., 2007; Zhu et al., 2014). Finally, due to their universal expansion in nearly all inflammatory conditions, independently of their etiology and physiology, MDSC have been found accumulated also after cellular, tissue or organs transplantation (Dilek et al., 2010; Ochando and Chen, 2012) as well as in autoimmune and chronic inflammatory diseases, such as multiple sclerosis, inflammatory bowel diseases, rheumatoid arthritis, type 1 diabetes or autoimmune hepatitis (Baniyash et al., 2014; Cripps and Gorham, 2011; Kurkó et al., 2014; Serafini, 2013; Smith and Reynolds, 2014; Whitfield-Larry et al., 2014). Several studies in rodent experimental models have demonstrated the importance of these immunosuppressive cells to keep immune responses in check in these aforementioned diseases. A too rapid interpretation of this would lead to the idea of using therapeutic strategies promoting MDSC accumulation and/or preventing their differentiation into mature cells to control undesired immune responses in transplantation, autoimmune or chronic inflammatory diseases. However, conflicting results emerged recently: while MDSC induced during acute inflammatory phases (those studied in experimental rodent models) are clearly beneficial, MDSC found in chronic inflammatory settings might not share similar characteristics. In fact, in contrast to cancer, injured or infected environment, MDSC isolated from autoimmune or chronic inflammatory environments appear to fail inhibiting T cells *ex vivo,* and rather add to the inflammation and recruitment of MDSC (Cripps and Gorham, 2011). Similarly, in chronic inflammatory bowel diseases, MDSC found in the colon released high quantity of IL-1β and IL-6, which promote detrimental Th17-biased cytotoxic immune responses (Kurmaeva et al., 2014). In solid transplant, MDSC play a critical role at different stages of graft tolerance induction. It could be useful at some stages to decrease the presence of MDSC specially the Mo-MDSC into the graft in order to improve clinical outcomes (Hock et al., 2015).

Based on these numerous clinical associations and key role played by MDSC in these diverse pathologies independently of their etiology and physiology, pharmaceutical approaches modulating MDSC in order to prevent or promote their accumulation now represent a growing field of interest. However, to date, there is no therapeutic approach allowing efficient MDSC modulation without adverse secondary effects. In fact, contrary to the majority of other cellular populations, MDSC in humans do not express specific markers allowing simply direct and specific targeting of these cells. It was recently found out that chemotherapeutical drugs such as gemcitabine, 5-fluorouracil, docetaxel or 2-hydroxy acetophenone glycinate can actually induce apoptosis of MDSC (Apetoh et al., 2011; Vincent et al., 2010). However, their efficacy to eliminate MDSC appears limited and such drugs also induce undesired apoptosis of other cells and are therefore associated with known toxicities. Furthermore, this approach faces the problem that inflammation confers MDSC resistance to apoptosis (Hu et al., 2013; Ostrand-Rosenberg et al., 2012).

Developing strategies to control MDSC immunosuppressive functions is made difficult by the multiplicity of suppressive mechanisms of action (iNos, Arg1, ROS, RNS, nutrient depletion, Fas-induced cell death, immunosuppressive cytokine secretion, immune deviation, induction of Treg, ...). In addition, these mechanisms are different depending of the type of MDSC (pro-myelocytic, Mo-MDSC or PMN-MDSC). The mechanisms at play is also dependent of the type of targeted immune (T and B lymphocyte, NK cells, macrophages, dendritic cells) or non-immune cells (e.g. vessel cells when increasing angiogenesis or cancer cells when promoting metastasis). It has been tried to prevent migration of MDSC (Highfill et al., 2014) to the tumor site (e.g. CXCR2 antagonist) but MDSC still continue to exercise their non-specific general immunosuppressive function on the periphery. Furthermore, since CXCR2 was express only by PMN-MDSC, but not Mo-MDSC, this approach will leave intact these Mo-MDSC which were described to be the more potent suppressive MDSC and to replenish PMN-MDSC as discuss above. To prevent induction and generation of MDSC, it has also been tried to block factors release by tumors or inflammation, that were identified as inductors of MDSC. An example is prostaglandin-E2 (PEG2) antagonists (Mao et al., 2014). However, these strategies will face with numerous other factors (e.g. GM-CSF, M-CSF, IL-6, IL-1β, IL-13, S100A8/A9 etc) responsible of MDSC induction.

Another concept is to target MDSC to induce their conversion into mature cells. This offers the advantage to convert detrimental immune cells (MDSC) into effector cells (macrophages and dendritic cells for Mo-MDSC and granulocytes for PMN-MDSC). For example, all-trans-retinoic acid (ATRA), a metabolite of vitamin A, could neutralize high ROS production and has potential to convert MDSC into mature granulocytes (Nefedova et al., 2007). In association with GM-CSF, ATRA induces macrophages and dendritic cells (Gabrilovich et al., 2001). However, these studies were performed in mice and when evaluated in humans, ATRA had an heterogeneous absorption and clearance among individuals and did not have any effect on either phenotype or function of mature myeloid cells (Mirza et al., 2006). Similarly, Vitamin D3 has been shown to decrease levels of immature myeloids cells *in vitro* and inducing their maturation. However its effect was more moderate than ATRA. DNA fragments that contain a high frequency of unmethylated deoxycytosine-deoxyguanine dinucleotide (CpG) motifs can stimulate immune cells *via* Toll-like receptor 9 (TLR9). Local administration of CPG in mice, for example in the tumor, was described to decrease PMN-MDSC (but not Mo-MDSC), to modestly alter their phenotype, and to reduce but not abolish their suppressive function (James et al., 2014). However, a previous study demonstrated that CpG induces the differentiation of bone marrow precursors into MDSC, limiting therefore its potential *in vivo* to reduce MDSC level (Chen et al., 2013).

As described herein, the inventors found out that MDSC could differentiate into a novel and unexpected population of non-suppressive lymphoid cells having a cytotoxic NK cell phenotype, different from macrophages, dendritic cells or granulocytes. They also identified that the signal regulatory protein alpha (SIRPa) tightly controls this previously unidentified MDSC road of differentiation. A therapeutic composition aimed at promoting this novel differentiation pathway will definitively have the potential to solve the medical problem posed by MDSC accumulation and associated immunodepression/ immunomodulation in diverse pathological settings. By reducing pathogenic accumulated MDSC and differentiating MDSC into non-suppressive effector cells, such therapeutic compositions have strong potential to synergize with current immunotherapies, chemotherapies and vaccination strategies which require proficient immune responses, in particular NK functions.

Signal regulatory protein alpha, or SIRPa (also termed CD172a or SHPS-1) was first identified as a membrane protein present mainly on macrophages and myeloid cells that was associated with the Src homology region 2 (SH2) domain-containing phosphatases-SHP-1 and SHP-2. SIRPa is the prototypic member of the SIRP paired receptor family of closely related SIRP proteins. Engagement of SIRPa by CD47 provides a downregulatory signal that inhibits host cell phagocytosis, and CD47 therefore functions as a "don't-eat-me" signal.

SIRPa is expressed on monocytes, most subpopulations of tissue macrophages, granulocytes, subsets of dendritic cells (DCs) in (lymphoid) tissues, some bone marrow progenitor cells, and to varying levels on neurons, with a notably high expression in synapse-rich areas of the brain, such as the granular layer of the cerebellum and the hippocampus (Seiffert et al, 1994; Adams et al. 1998; Milling et al, 2010).

The SIRPa interaction with CD47 is largely described and provides a downregulatory signal that inhibits host cell phagocytosis (see review Barclay et al, Annu. Rev. Immunol., 2014). Both CD47 and SIRPa also engage in other interactions. Investigators have suggested that the lung surfactant proteins SP-A and SP-D control inflammatory responses in the lung through interactions with SIRPa (Janssen et al, 2008).

One of the best characterized physiological functions of CD47-SIRPa interactions is their role in the homeostasis of hematopoietic cells, in particular red blood cells and platelets. Because CD47 serves as a don't-eat-me signal and, as such, is an important determinant of host cell phagocytosis by macrophages, the potential contribution of CD47-SIRPa interactions in cancer cell clearance has been intensely investigated in recent years.

The SIRPa/CD47 pathway is nowadays also subject to different pharmaceutical developments to enhance macrophages phagocytosis. In fact, like infected cells, cancer cells carry aberrant cargo such as unfamiliar proteins or normal proteins at abnormal levels, yet these cells frequently subvert innate immune control mechanisms by concurrently over-expressing immunoregulatory molecules. It is becoming increasingly clear that one such mechanism involves CD47 (Barclay and Van den Berg, 2014), a protein of "self" expressed by normal cells. CD47 interacts with SIRPa. This leads to the transmission of a "don't eat me" signal to phagocytic macrophages, which then leave target cells unaffected (Oldenborg et al., 2000). Over-expression of CD47 by cancer cells renders them resistant to macrophages, even when the cancer cells are coated with therapeutic antibodies (Zhao et al., 2011), and correlates with poor clinical outcomes in numerous solid and hematological cancers (Majeti et al., 2009; Willingham et al., 2012). In experimental models, in particular human tumor-xenograft models in immunodeficient mice, blockade of the CD47/SIRPa pathway was very effective to promote tumor elimination by macrophages and to decrease cancer cell dissemination and metastasis formation (Chao et al., 2011; Edris et al., 2012; Uluçkan et al., 2009; Wang et al., 2013). In these studies, MDSC function or phenotype has not been studied. Blockade of the CD47/SIRPa pathway, by enhancing antibody-dependent phagocytosis by macrophages, has been described to synergize with depleting therapeutic anticancer antibodies (Weiskopf et al., 2013) such as Trastuzumab (anti-Her2), Cetuximab (anti-EGFR), Rituximab (anti-CD20) and Alemtuzumab (anti-CD52).

One set of divisions which are becoming increasingly "blurred" are those between T lymphocytes, Natural Killer (NK) cells, and NK-T cells. These subsets share common expression of specific molecules, including the C type lectin CD161. This surface molecule was originally identified as the human homolog of the NKRP1 glycoproteins expressed on rodent NK cells, demonstrating 46- 47% homology with its rodent counter parts. Human NKRP1A, or CD161, is composed of a disulfide-linked homodimer of about 40 kDa subunits. It is expressed by the majority of NK cells and approximately 24% of peripheral T cells (Lanier etal., 1994), including both γδ and αβ TCR expressing subsets (Maggietal., 2010) and NK-T cells. As NK-T cells compose less than 1% of human peripheral blood T cells (Gumperzetal., 2002), CD161+T cells must represent a distinct lineage of T lymphocytes (Takahashi et al., 2006).

As mentioned above, SIRPa has been described to regulate the phagocytic function of myeloid cells, the antigen presentation and cytokine secretion of dendritic cells and trafficking of mature granulocytes. However, the function of SIRPa on suppressive function of MDSC has never been disclosed. Dugast et al. (2008) showed for the first time the expression of SIRPa on rat MDSC in a kidney allotransplantation model. However, they did not identify any role of the SIRPa/CD47 pathway in MDSC biology, nor did they report any suppressive activity by using anti-SIRPa antibody. Hence, this document does not show nor suggest that the inhibition of SIRPa pathway on MDSC could differentiate MDSC cells into non-suppressive cells as disclosed herein.

WO2010/130053 disclosed a method for treating hematological cancer comprising modulating the interaction between human SIRPa and CD47. This document showed that the blockade of SIRPa-CD47 induces the activation of the innate immune system via the phagocytosis pathway. In the transplantation model of human leukemia myeloid cells used this patent application, the transplant was rejected when animals were treated with an antagonist of CD47. This result suggests an increase of phagocytosis upon treatment with anti-CD47, but not an inhibition of suppressive activity of MDSC and/or a differentiation of MDSC into non suppressive cells. WO2013/056352 also discloses a method for treating a particular subtype of hematological cancer, namely acute myeloid leukemia, a liquid cancer which is not related to solid cancer.

A method for inhibiting cell functioning for use in anti-inflammatory and anti-tumor therapies was described in WO0066159. This method comprises administering a drug comprising a substance that specifically recognizes the extracellular domain of SIRP and that inhibits the functioning of pathologic myeloid cells. Given example of myeloid cells are macrophages, most of anti-SIRPa described in documents aim at blocking macrophages activation and inhibiting phagocytosis. This method does not suggest any advantageous action of anti-SIRPa molecules on normal myeloid cells and more specifically MDSC.

A first aspect of the present invention is hence the use of a compound, as defined in the claims, blocking the interaction between the signal regulatory protein alpha (SIRPa) and CD47, for treating solid cancer in a patient, the solid cancer being selected from the group consisting of carcinoma, adenocarcinoma, breast cancer, prostate cancer, ovarian cancer, lung cancer, pancreatic cancer, colon cancer, sarcoma, lymphoma and blastoma.

Among the compounds which can be used according to the present invention, one can cite antibodies and fragments thereof, especially anti-SIRP antibodies such as those used in the experiments described below, or any other blocking antibody selected amongst the many anti-SIRPa commercially available antibodies) or fragments of antibodies.

According to a particular embodiment of the above use, the compound blocking the interaction between SIRPa and CD47 is used for differentiating monocytic MDSC (Mo-MDSC) into non suppressive cells.

According to another particular embodiment of the above use, the compound blocking the interaction between SIRPa and CD47 is used for differentiating MDSC into non suppressive lymphoid cells, preferably into effector lymphoid cells.

The compound blocking the interaction between SIRPa CD47 is preferably chosen so that the non suppressive cells obtained by differentiation of MDSC are negative for MHC Class II and positive for at least one marker of natural killer (NK) cells. A non-limitative list of markers of NK cells is provided in the following table.

**Table 1: Non exhaustive list of NK markers**

| Rat | Mice | Human |
|---|---|---|
| CD161 (NKRP1) | CD161 (NKRP1) | CD161 (NKRP1) |
| | CD335 (NKp46) | CD335 (NKp46) |
| | CD122 (IL2Rbeta) | CD122 (IL2Rbeta) |
| CD94 (NKG2) | CD94 (NKG2) | CD94 (NKG2) |
| | CD314 (NKG2D) | CD314 (NKG2D) |
| Ly49 family | Ly49 family | CD336 (NKp44) |
| NKG2A family | NKG2A family | CD337 (NKp30) |
| | CD49b | CD158 (KIR family) |
| | | CD16 (FcgIIIA) |
| | | CD56 |
| | | CD57 |

As mentioned above, MDSC-induced immunodepression plays an important and deleterious role in many diseases and conditions. Among the conditions susceptible of being improved or prevented by differentiating myeloid-derived suppressor cells (MDSC) into non suppressive cells, one can cite solid cancers.

The compound blocking the interaction between SIRPa and CD47 is used for treating a patient having a solid cancer. As used herein, "cancer" means all types of cancers. Non limitative examples of solid cancers are carcinomas or adenocarcinomas such as breast, prostate, ovary, lung, pancreas or colon cancer, sarcomas, lymphomas, and blastomas. As used herein, the terms "treat", "treatment" and "treating" refer to any reduction or amelioration of the progression, severity, and/or duration of solid cancer, particularly a solid tumor; for example in a breast cancer, reduction of one or more symptoms thereof that results from the administration of one or more therapies. The treatment by a compound blocking the interaction between SIRPa and CD47 can be administered together with any other antineoplastic treatment, such as surgery, chemotherapy, biological therapy, immunological therapy, *etc.* In case of a coadministration, the beneficial effect of the compound blocking the interaction between SIRPa and CD47 is measured by comparing the efficiency of the combined treatment to that classically obtained with the same treatment but without said compound.

According to another particular embodiment of the method according to the invention, the compound blocking the interaction between SIRPa and CD47 is used for treating a metastatic cancer.

Another aspect of the present invention is thus the use of a compound, as defined in the claims, blocking the interaction between SIRPa and CD47, in combination with a second therapeutic agent, to treat a solid cancer in an individual in need thereof. According to preferred embodiments of this aspect of the present invention, the second therapeutic agent is selected from the group consisting of chemotherapeutic agents, radiotherapy, surgery, immunotherapeutic agents, antibiotics and probiotics.

In particular, the second therapeutic agent can advantageously be selected from the group consisting of therapeutic vaccines and immune checkpoint blockers or activators such as, for example, anti-PDL1, anti-PD1 anti-CTLA4 and anti-CD137. As exemplified in the experimental part below, these combinations produce synergistic effects.

The skilled artisan will chose an appropriate sample to perform this follow-up method, depending on the situation. For example, when the treatment is administered to a patient suffering from a solid cancer, a sample from the tumor will advantageously be used. A blood sample can also be used, in the same situation but also in other situations, as well as y tissue sample, sample of synovial fluid, *etc..*

Other characteristics of the invention will also become apparent in the course of the description which follows of the experiments and biological assays which have been conducted in the framework of the invention and which provide it with the required experimental support.

### Figures legends

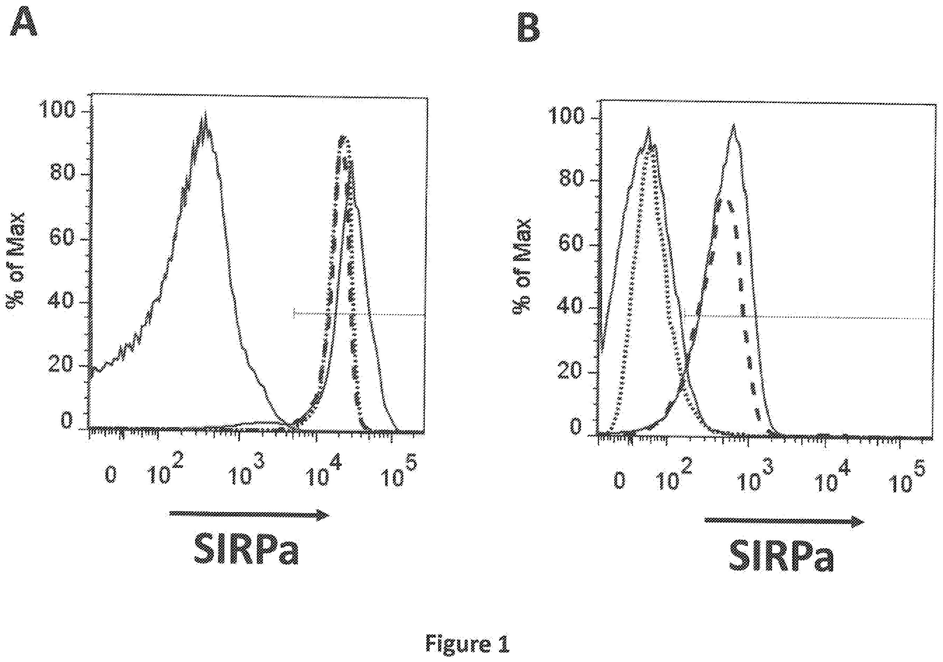

Figure 1: Mice and human MDSC express SIRPa
   A/ Freshly isolated mouse spleen cells were stained with a fluorescent anti-mouse SIRPa monoclonal antibody and analyzed by flow cytometry. Cells were sub-divided according to the following phenotype: CD11b⁻ cells (gray histogram), CD11b⁺ MHC Class II⁺ cells (solid line), CD11b⁺ MHC Class II⁻ Ly6C^{high} Ly6G⁻ cells (Mo-MDSC; dashed line) and CD11b⁺ MHC Class II⁻ Ly6C⁺ Ly6G⁺ cells (PMN-MDSC; dotted line). B/ Freshly isolated human peripheral blood mononuclear cells from healthy volunteers were stained with a fluorescent anti-human SIRPa monoclonal antibody and analyzed by flow cytometry. Cells were sub-divided according to the following phenotype: CD11b⁻ cells (grey histogram), CD11b⁺ HLA-DR⁺ cells (solid line), CD11b⁺ HLA-DR^{-/low} CD33⁺ CD14⁺ CD15⁻ cells (Mo-MDSC; dashed line) and CD11b⁺ MHC HLA-DR⁻ CD33⁺ CD14⁻ CD15⁺ cells (PMN-MDSC; dotted line).
Figure 2: Anti-SIRPa mAb induces human Mo-MDSC phenotype change after two days of culture
   Phenotype of human Mo-MDSC (CD11b⁺ HLA-DR^{-/low} CD33⁺ CD14⁺ CD15⁻ cells) for CD161 and CD11c expression directly after flow cytometry sorting (left) or after two days of culture with a control irrelevant monoclonal antibody or anti-SIRPa monoclonal antibody.
Figure 3: Anti-SIRPa mAb induces rat MDSC phenotype change after two days of culture
   MDSC (CD11b⁺ MHC Class II⁻ NKRP1^{low} spleen cells) phenotype for the indicated markers directly after flow cytometry sorting (grey histogram) or after two days of culture with a control irrelevant monoclonal antibody (dotted line) or anti-SIRPa monoclonal antibody (solid line).
Figure 4: Anti-SIRPa mAb -induced differentiation overcomes GM-CSF-induced differentiation
   Rat MDSC (CD11b⁺ MHC Class II⁻ NKRP1^{low} spleen cells) phenotype for MHC Class II and CD103 (top) or CD11b and CD80 (bottom) after two days of culture with a control irrelevant monoclonal antibody (dotted line) or anti-SIRPa monoclonal antibody (solid line), with or without addition of GM-CSF.
Figure 5: Anti-SIRPa mAb -induced differentiated MDSC loss immunosuppressive functions
   A/ T-lymphocyte proliferation in the presence of the indicated ratio of freshly purified MDSC (undifferenciated) and a control irrelevant monoclonal antibody (white bars) or anti-SIRPa monoclonal antibody (black bars). B/ T-lymphocyte proliferation in the presence of the indicated ratio of two-days cultured MDSC with a control irrelevant monoclonal antibody (white bars) or anti-SIRPa monoclonal antibody (black bars). No additional antibody was added during the proliferation assay with differenciated MDSC.

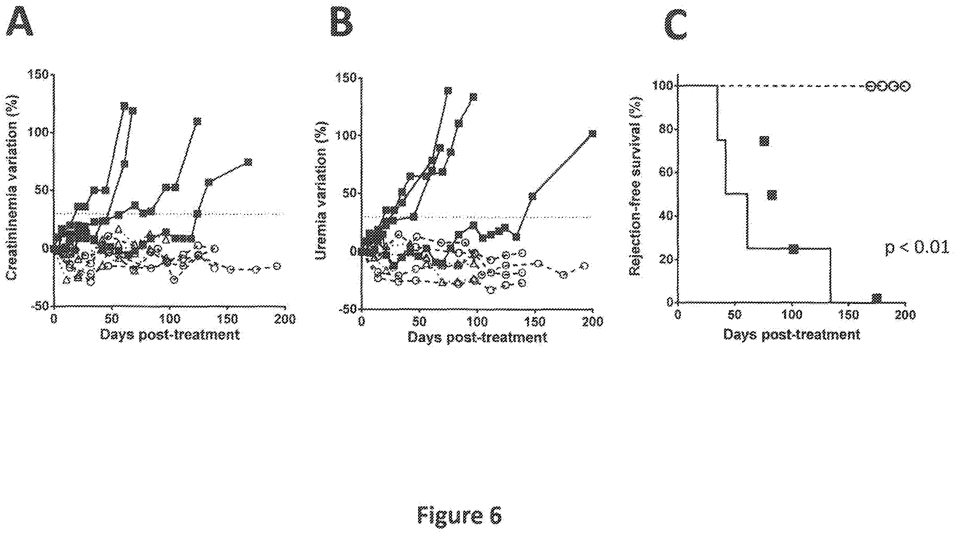

Figure 6: Anti-SIRPa mAb treatment breaks MDSC-dependent immune tolerance
   Percentage variation of Creatininemia (A) and Uremia (B) from day 0 to 200 and C/ Rejection-free survival of tolerant rat kidney allograft recipients treated with an irrelevant control antibody (open round, n=4) or anti-SIRPa monoclonal antibody (black square, n=4). Dotted line in A and B represent a 30% of variation threshold, above which animals were considered at rejection.

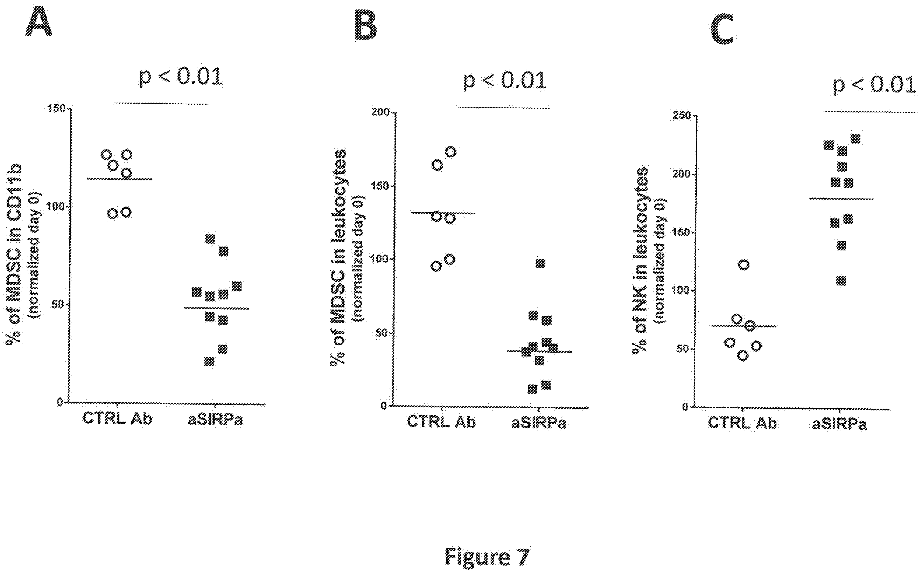

Figure 7: Anti-SIRPa mAb treatment decrease MDSC and increase NK cells in periphery
   Variation percentage from day 0 of MDSC in myeloid cells (A) or total leukocytes (B) of tolerated kidney allograft recipients and naïve rats after an average ten days of treatment with an irrelevant control antibody (open round) or anti-SIRPa monoclonal antibody (black square). C/ Same as in B, for variation percentage from day 0 of NK cells (CD161^{high}) in total leukocytes.

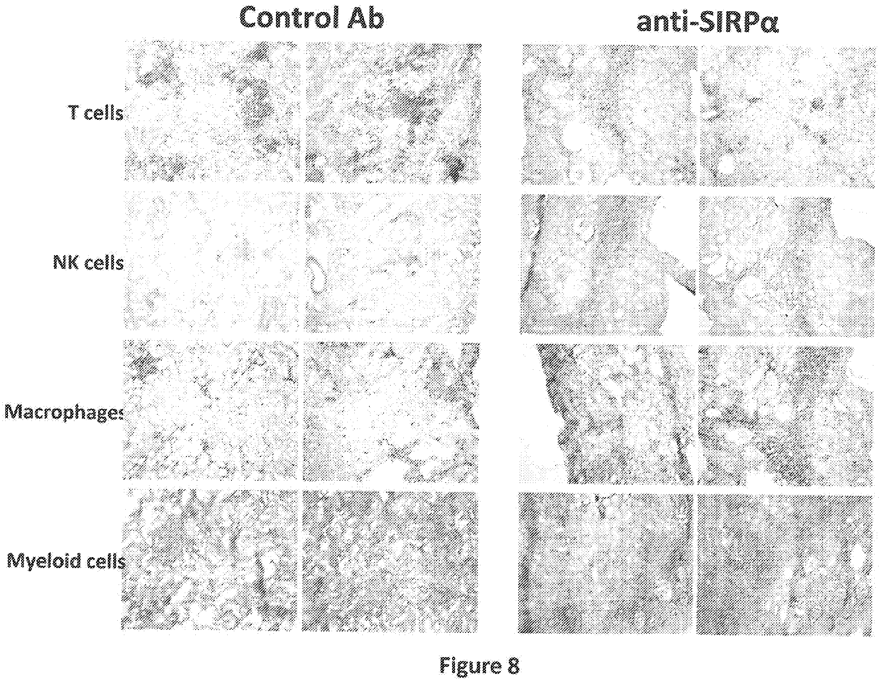

Figure 8: Anti-SIRPa mAb treatment induces NK cells and macrophages infiltration
   Graft immunohistology staining of T lymphocytes (TCRαβ⁺), NK cells (CD161⁺), macrophages (CD68⁺) and myeloid cells (CD11b/c⁺) of tolerated kidney allograft recipient treated with an irrelevant control antibody (left) or anti-SIRPa monoclonal antibody (right).

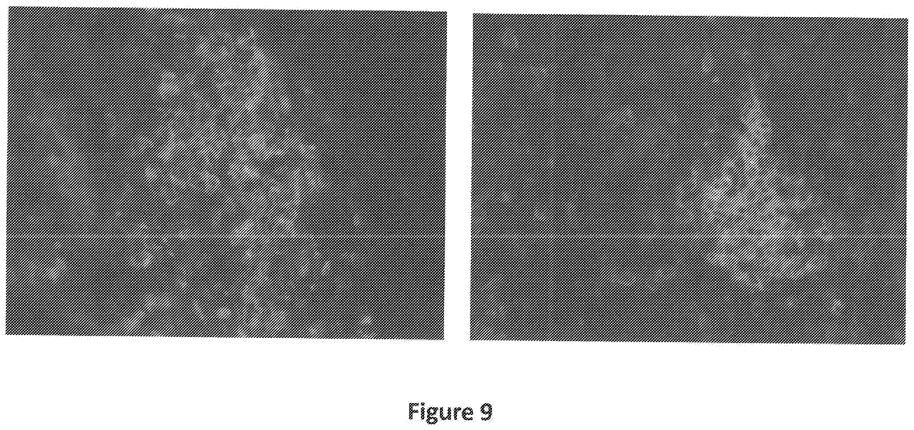

Figure 9: Anti-SIRPa mAb treatment reduce regulator T cells infiltration.
   Graft immunohistology staining of T lymphocytes (TCRαβ⁺) and regulatory T cells (TCRαβ⁺Foxp3⁺) of tolerated kidney allograft recipient treated with an irrelevant control antibody (left) or anti-SIRPa monoclonal antibody (right).

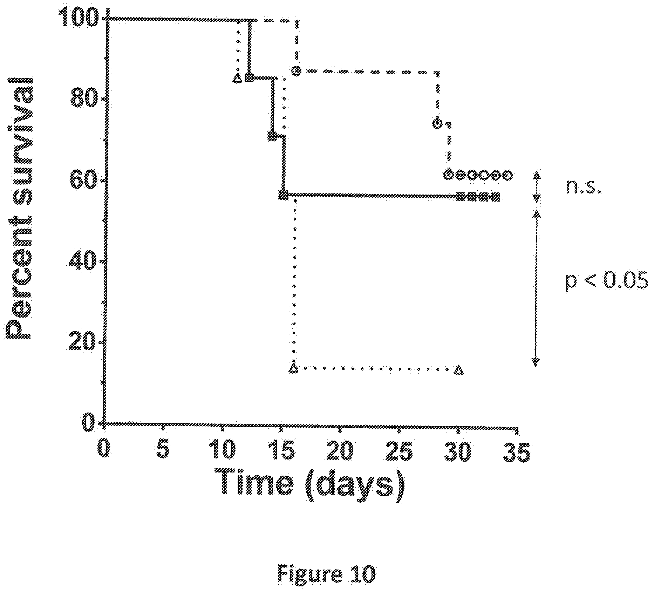

Figure 10: Anti-SIRPa mAb treatment prolong survival in a hepatocellular carcinoma cancer model.
   Overall survival of mice inoculated with 2.5 x 10⁶ Hepa1.6 mouse hepatoma cells through the portal vein and treated either with an irrelevant control antibody (dotted line) or anti-SIRPa monoclonal antibody (solid line) 3 times per week, or daily with oral gavage of Sorafenib (dashed line) as standard of care control.

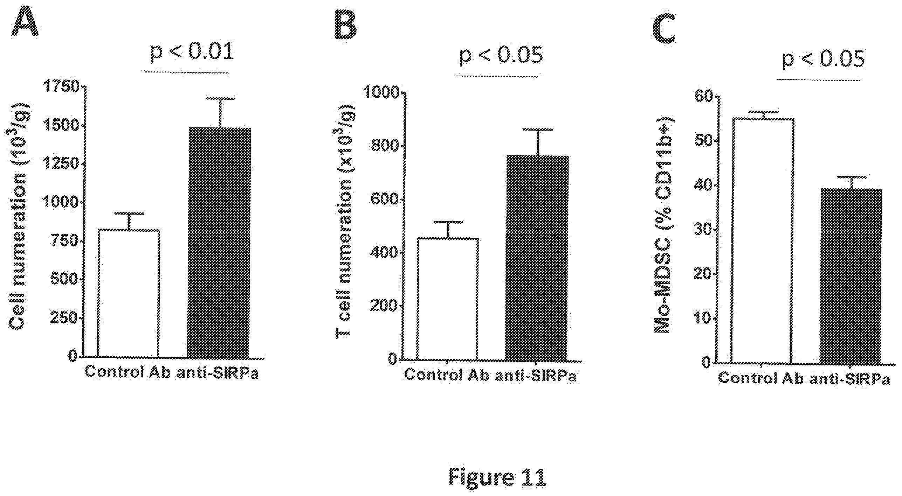

Figure 11: Anti-SIRPa mAb induces tumor leukocytes recruitment while reduces MDSC in hepatocellular carcinoma cancer model.
   Mice inoculated with 2.5 x 10⁶ Hepa1.6 tumor cells through the portal vein were sacrificed at two weeks after tumor inoculation. Liver non-parenchymal cells (NPC) were extracted, counted and analyzed by flow cytometry. A/ Number of liver NPC extracted, B/ Number of T-lymphocytes (CD3⁺) in liver NPC and C/ percentage of Mo-MDSC (CD11b⁺ MHC Class II⁻ Ly6C^{high} Ly6G⁻) in CD11b⁺ myeloid cells of mice treated with an irrelevant control antibody (white bars, n=7) or anti-SIRPa monoclonal antibody (black bars, n=7) 3 times per week.

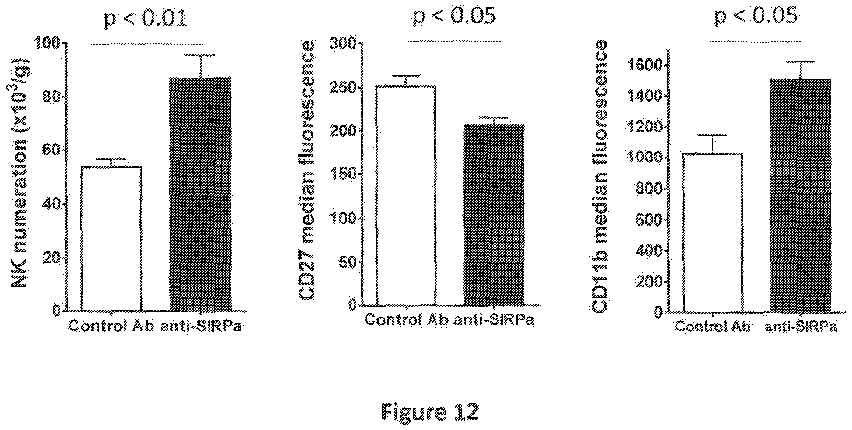

Figure 12: Anti-SIRPa mAb induces mature NK cells accumulation in hepatocellular carcinoma model.
   Mice inoculated with 2.5 x 10⁶ Hepa1.6 tumor cells through the portal vein were sacrificed at two weeks after tumor inoculation. Liver non-parenchymal cells (NPC) were extracted, counted and analyzed by flow cytometry. A/ Number of NK cells (CD161⁺) extracted cells in liver NPC extracted from mice treated with an irrelevant control antibody (white bars, n=7) or anti-SIRPa monoclonal antibody (black bars, n=7) 3 times per week. B/ CD27 (immature NK marker) and CD11b (mature NK marker) expression of liver NPC NK cells.

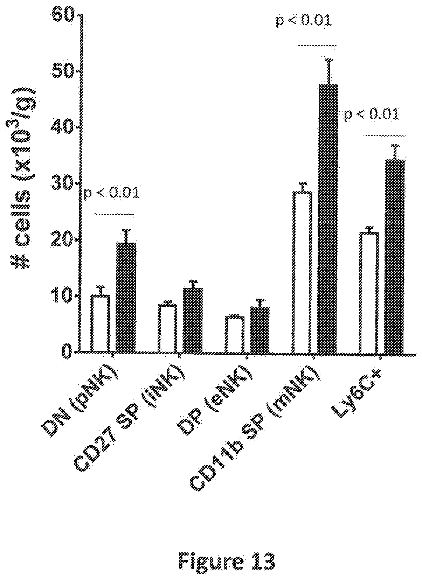

Figure 13: Anti-SIRPa mAb induces mature NK cells accumulation in hepatocellular carcinoma model.
   Mice inoculated with 2.5 x 10⁶ Hepa1.6 tumor cells through the portal vein were sacrificed at two weeks after tumor inoculation. Liver non-parenchymal cells (NPC) were extracted, counted and analyzed by flow cytometry. Number of NK cells sub-populations in liver NPC as follows: double-negative (DN) for CD11b and CD27 (pNK: precursor NK); CD27 single (CD27 SP) positive (iNK: immature NK); double positive for CD27 and CD11b (eNK: effector NK); CD11b single (CD11b SP) positive (mNK: mature NK); and Ly6C⁺ NK cells.

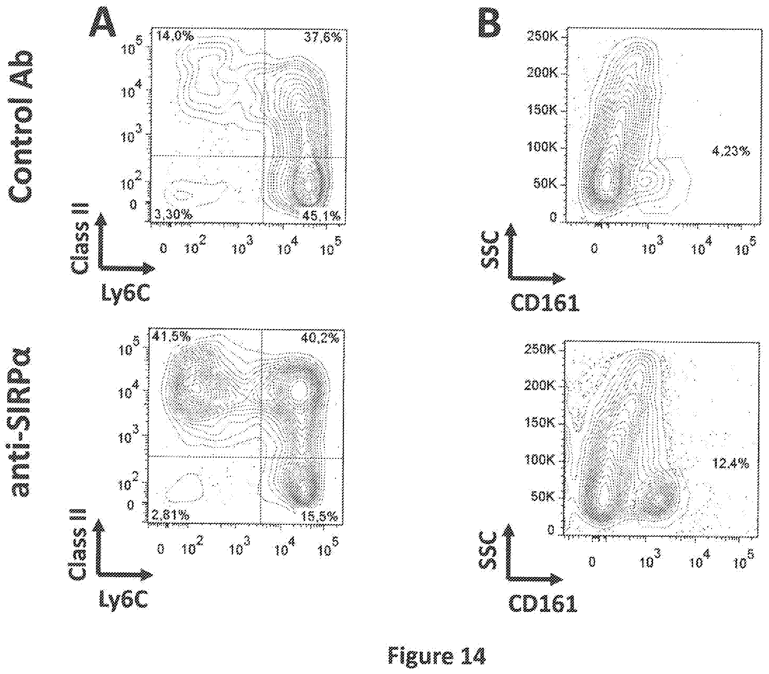

Figure 14: Anti-SIRPa mAb reduces number of MDSC and increase tumor-infiltrating NK cells in the tumor in melanoma model.
   Mice injected subcutaneously with 2x 10⁶ B16 melanoma cells and treated with an irrelevant control antibody (top) or anti-SIRPa monoclonal antibody (bottom) 3 times per week were sacrificed at two weeks after tumor inoculation. Tumor leukocytes infiltrating cells were extracted and the proportion of (A) MDSC (CD11b⁺ Class II⁻ Ly6C^{high}) and the proportion of (B) NK cells (CD161⁺) were analyzed by flow cytometry. Results are representative of 5 mice in each condition.

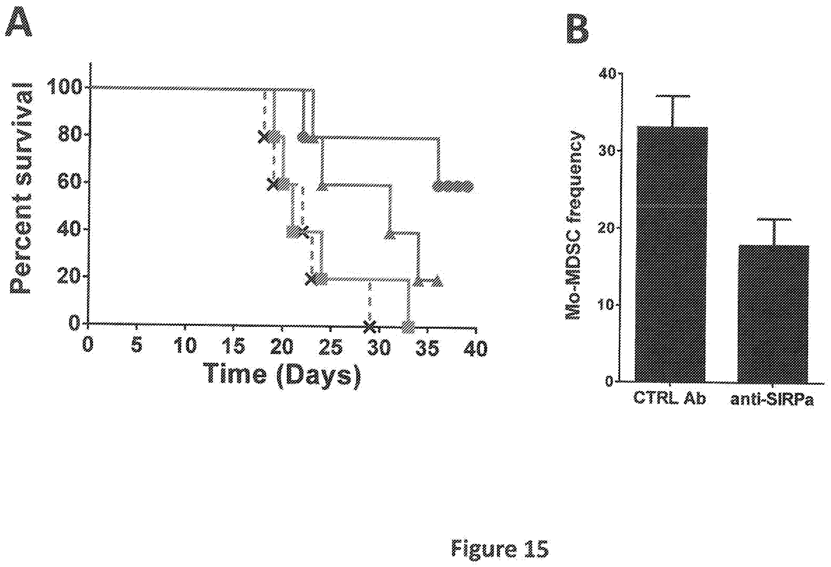

Figure 15: Anti-SIRPa mAb significatively increases the survival of mice co-treated with anti-PDL1 antibody and reduces number of Mo-MDSC in an in vivo melanoma model.
   In the same time as tumor inoculation, animals were treated with isotype control antibody (Iso ctrl: star: n=5), or an anti-Sirpα antibody (p84 clone: square; n= 5) or an anti-PD-L1 antibody (10F-9G2 mAb: triangle; n=8) or a combined treatment (anti-Sirpα+anti-PD-L1: circle; n=5). A. The overall survival rate was then analyzed. B. Some animals were sacrificed 2 weeks after first inoculation, animals treated with irrelevant antibody were compared to animals receiving anti-SIRPa antibody. Tumor leukocytes infiltrating cells were extracted and the proportion of Mo-MDSC markers was analyzed by FACS using CD11b⁺ Class II⁻ Ly6C^{high}.

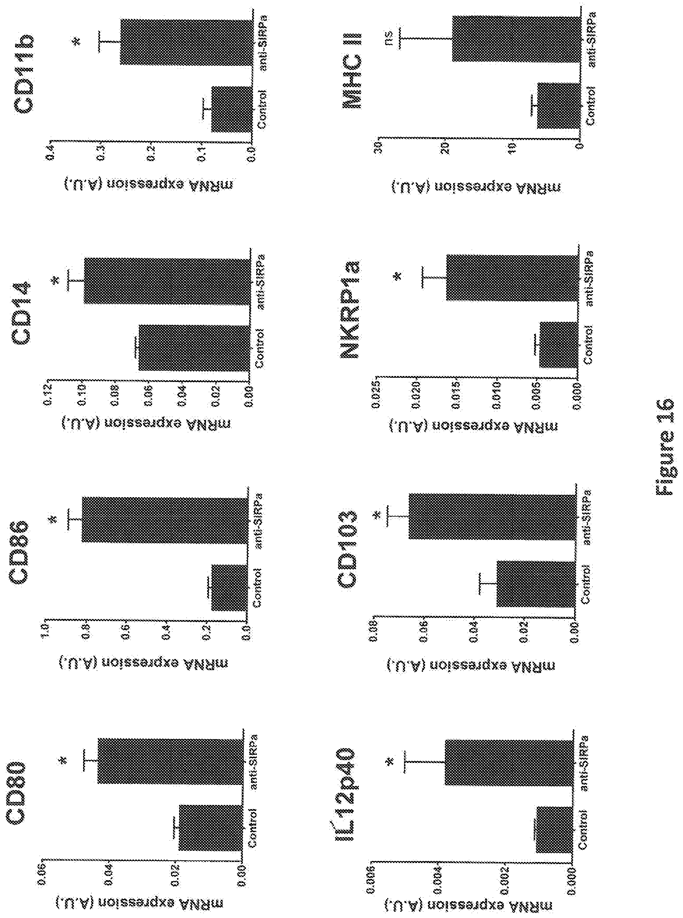

Figure 16: Anti-SIRPa treatment modify the mRNA expression of cell surface markers of infiltrating cells after breaking the immune tolerance in a model of rat kidney transplant.
   mRNA expression of CD80, CD86, CD14, CD11b, IL12p40, CD103, NKRP1, MHCII were measured and are increased after anti-SIRPa treatment compared to tolerant control.

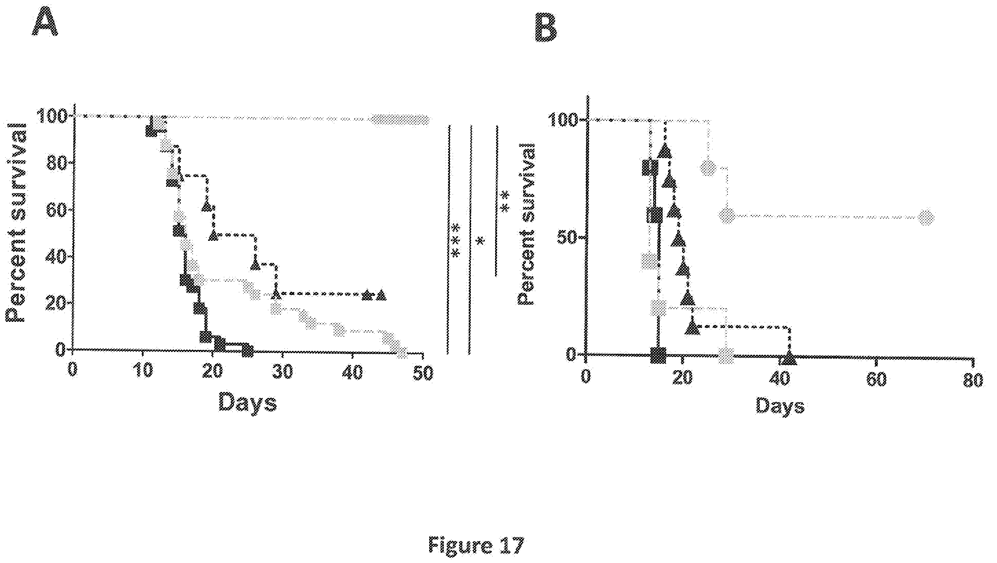

Figure 17 : Effect of a single anti-Sirpα treatment or combined immunomodulatory treatment on an in vivo model of Hepatocarcinoma:
   One week after tumor inoculation, animals were treated 3 times/week for 4 weeks A. with isotype control antibody (Iso ctrl: black square: n=33), or an anti-Sirpα antibody (p84 clone: grey square; n= 33) or an antiCD137 antibody (4-1BB mAb: black triangle; n=8) or a combined treatment (Anti-Sirpα+anti-CD137: grey diamond; n=8). The overall survival rate was then analyzed. B. animals were treated 2 times/week for 4 weeks with isotype control antibody (Iso ctrl: black square: n=5), or an anti-Sirpα antibody (p84 clone: grey square; n= 5) or an anti-PD-L1 antibody (10F-9G2 mAb: black triangle; n=8) or a combined treatment (anti-Sirpα+anti-PD-L1: grey diamond; n=5). The overall survival rate was then analyzed.

### EXAMPLES

### 1. Materials and Methods

### MDSC phenotype

Human blood MDSC were characterized and sorted by flow cytometry as follow: CD11b⁺ CD33⁺ HLA-DR^{low/-} CD14⁺ CD15⁻for Mo-MDSC and CD11b⁺ CD33⁺ HLA-DR⁻ CD14⁻ CD15⁺ for PMN-MDSC. Rat blood and spleen MDSC were characterized and sorted by flow cytometry as CD11b⁺ MHC ClassII⁻NKRP1^{int} (both Mo-MDSC and PMN-MDSC was included in this phenotype). Mouse MDSC were characterize as follow: CD11b⁺ MHC Class II⁻ Ly6C^{high} Ly6G⁻ for Mo-MDSC and CD11b⁺ MHC Class II⁻ Ly6C⁺ Ly6G⁺ for PMN-MDSC.

### MDSC purification and culture

MDSC were purified by flow cytometry according to previously described phenotypes. Purity of cells sorted by flow cytometry was higher than 99%. Freshly purified MDSC were then seeded at 50 × 10³ cells/well in 96-well flat-bottomed microtiter plates and cultured for two days in RPMI-1640 medium (supplemented with 2 mM L-glutamine, 100 U/ml penicillin, 0.1 mg/ml streptomycin, 10% heat-inactivated fetal calf serum, 1% nonessential amino acids, 5 mM HEPES, 1 mM sodium pyruvate, and 1 µM 2-mercaptoethanol) and with irrelevant control antibody or anti-SIRPa monoclonal antibody (clone SE7C2 (Santa Cruz Biotechnology) and SE5A5 (Biolegend) for human MDSC or clone ED9 (AbD Serotec) for rat MDSC) at 10 µg/ml. In a different setting, recombinant GM-CSF (10 ng/ml) was also added to force the macrophages/dendritic cells differentiation. After two days, supernatant was harvested and cells were remove by incubating with 2mM EDTA for 5 min in 37°C. Cells were then stained with fluorescent antibody to characterize their phenotype by flow cytometry. In parallel, cells were resuspended in culture medium to assess their immunosuppressive function on T-lymphocyte proliferation.

### MDSC-immunosuppressive function assay

Flat-bottom 96-well plates were coated with anti-CD3 antibodies (0.5µg/ml; 2 hours at 37°C). Rat spleen cells were seeded at 50 × 10³ cells/well in triplicate and cultured for 3 days in RPMI-1640 medium (supplemented with 2 mM L-glutamine, 100 U/ml penicillin, 0.1 mg/ml streptomycin, 10% heat-inactivated fetal calf serum, 1% nonessential amino acids, 5 mM HEPES, 1 mM sodium pyruvate, and 1 µM 2-mercaptoethanol) with 2 µg/ml of anti-CD28 monoclonal antibodies. Different ratio of freshly isolated MDSC was added on day 0 of culture on these polyclonaly-activated T lymphocytes culture. Control antibody or anti-SIRPa antibody (SE7C2, Santa Cruz Biotechnology^{®}, inc. or SE5A5, Biolegend^{®}) was added at 10 µg/ml from day 0 of culture. In other plates, Mo-MDSC maintained for 48h in the presence of control or anti-SIRPa antibodies were washed to remove antibodies and added at different ratios in a similar suppression assay, without addition of new antibodies. Proliferation was measured at day 3 by addition of 0.5 µCi[³H]thymidine per well.

### Kidney allograft tolerance rat model

Seven- to nine-week-old male Lewis.1W (RT1u) and Lewis.1A (RT1a) congenic rats were obtained from Janvier (Savigny/Orges, France) and maintained in our animal facility under specific pathogen-free conditions, according to institutional guidelines. Kidney allografts were performed, as previously described (Dugast et al., 2008). One native Lewis. 1W kidney (right side) was replaced by the LEW.1A donor allograft, and a contralateral nephrectomy was performed 7 d later, after which time the recipient's survival depended on the proper functioning of the allograft. These rats were treated by an anti-CD28 antibody (hybridoma JJ319) i.p. at 0.3 mg/day for 7 days, starting on the day of transplantation. Without treatment, the grafts were rejected 11 days after transplantation. Animals were considered as tolerant when no rejection occurred within 100 days. Tolerant recipient were then treated i.p. with 500µg of anti-SIRPa antibody (clone ED9, AbDSerotec) or irrelevant control antibody (clone 3G8) on day 150 and then 300µg twice/week during 21 days. Blood samples were drawn for flow cytometry analyses. Creatininemia and uremia was monitored in sera. Animals were sacrificed when renal graft dysfunction was evidenced by a 2-fold increase from day 0 of either creatininemia or uremia.

### Immunohistochemical staining

Frozen sections (10mm) were prepared from renal biopsies. Slides were air dried at room temperature for 1 h before acetone fixation for 10 min at room temperature. Sections were saturated, and when required (i.g. Foxp3 staining) permeabilized with 0.5% saponin (Sigma-Aldrich, St. Louis, MO), in the saturated solution (PBS containing 5% rat serum, 2% normal goat serum, and 4% BSA). Sections were incubated overnight with primary antibody at 4°C, followed by fluorescent secondary antibody or biotinylated secondary antibody and colored with ABC Vectastainkit (Vector, Burlingame, CA) and diaminobenzidine (DAB) kit before eosin and hematoxylin coloration. Slides were analyzed using standard or fluorescence microscopy and AxioVision imaging software (Carl Zeiss, Le Pecq, France).

Primary antibody used were anti-rat TCRαβ (clone R73), CD161 (clone 3.2.3), CD68 (clone ED1), CD11b/c (clone OX42) and Foxp3 (clone FJK-16s).

### Quantitative real-time PCR (qPCR)

Quantitative real-time PCR was performed in an Applied Biosystems Viia7 system using SYBR Green and TaqMan PCR core reagents. RNA is extracted from kidney tissues homogenization in Trizol reagent, separated from DNA and proteins with chloroform, precipitated with isopropanol, washed with ethanol, dried and then resuspended in RNase-free water. After a DNAse treatment, RNA is retro-transcripted to obtain complementary DNA (cDNA). Relative gene expression was calculated with the 2(-dCt) method in comparison with the housekeeping gene HPRT. All samples were analyzed in duplicate. Expression of genes of interest was compared between tolerant animals treated with control antibody (3G8) or anti-SIRPa antibody (ED9).

**Table 2: Primers used in the quantitative PCR**

| Primers (rat) | Sense | Sequence from 5' to 3' | SEQ ID No |
|---|---|---|---|
| rHPRTFor | Forward | CCTTGGTCAAGCAGTACAGCC | 1 |
| rHPRTRev | Reverse | TTCGCTGATGACACAAACATGA | 2 |
| rCD14For | Forward | CAACAGGCTGGATAGGAAACCT | 3 |
| rCD14Rev | Reverse | TGACTACGCCAGAGTTATACGC | 4 |
| rCD86For | Forward | ACAGCAAAAGACACCCACGG | 5 |
| rCD86Rev | Reverse | CTTGTTTCATTCTGAGCCTCCTC | 6 |
| rIL12p40For | Forward | TCATCAGGGACATCATCAAACC | 7 |
| rIL12p40Rev | Reverse | CGAGGAACGCACCTTTCTG | 8 |
| rCD80For | Forward | GGCATTGCTGTCCTGTGATTAC | 9 |
| rCD80Rev | Reverse | GGAGTAGTTGTTAGCGATGTCGTA | 10 |

**Table 3: Primer/Probe sets used for TaqMan gene expression measurements**

| Target | ThermoFisher Primer/Probe set reference |
|---|---|
| CD103 (integrin alpha e) | Rn01460526_m1 |
| CD11b | Rn00709342_m1 |
| ClassII | Rn01429090_g1 |
| NKRP1 (CD161a) | Rn01749035_m1 |

### Hepatocellular carcinoma mice model

Eight-weeks-old C57Bl/6J male mice received 2.5 x 10⁶Hepa1.6 mouse hepatoma cells in 100 µL through the portal vein, as previously described (Gauttier et al., 2014). Four and eight days after tumour inoculation, mice were injected intraperitoneally with 100 µg of rat anti-CD137 mAb (4-1BB mAb), or with 300µg of anti-mouse SIRPa monoclonal antibody (clone P84 from Merck Millipore) or both antibodies or an irrelevant control antibody (clone 3G8) 3 times/week for 4 weeks (Figure 17A) or with 200 µg of the anti-PD-L1 mAb (clone 10F-9G2 from BioXCell) or received both antibodies (anti-Sirpa + anti-PDL1) for 4 weeks (Figure 17B). Sorafenib (Nexavar-Bayer) treated mice received daily 100µl oral gavage of 40mg/kg from day 0 to day 28 (Figure 10). The overall survival was analyzed. Some mice were sacrificed at day 14 after tumor inoculation for leukocytes tumor infiltration quantification and characterization by isolation of non parenchymal cells of the liver by a Percoll gradient and FACS analysis.

### Melanoma mice model

Eight-weeks-old C57Bl/6J male mice received subcutaneous injection of 2 x 10⁶ B16-Ova mouse melanoma cells into the flank. Mice were treated i.p. from day 0 after tumor inoculation with either 300µg of an irrelevant control antibody (clone 3G8) or anti-mouse SIRPa monoclonal antibody (clone P84) 3 times per week or with 200 µg of the anti-PD-L1 mAb (clone 10F-9G2 from BioXCell) twice a week or received both antibodies (anti-Sirpa and anti-PD-L1 antibodies) for 4 weeks. Some animals were sacrificed at two weeks after tumor inoculation to characterized tumor leukocytes infiltrates by flow cytometry. The overall survival was analyzed.

### 2. Results

### MDSC express SIRPa

The inventors previously described (Dugast et al., 2008) that rat MDSC (monocytic and granulocytic) express high level of SIRPa at their surface. Figure 1 now shows that mouse Mo-MDSC and PMN-MDSC also express SIRPa at their surface at similar level to mature myeloid cells (CD11b⁺ Class II⁺). In contrast, in human, SIRPa is expressed only on Mo-MDSC, at similar levels to mature myeloids cells (CD11b⁺ HLA-DR⁺), whereas PMN-MDSC do not express SIRPa.

### SIRPa controls the differentiation of Mo-MDSC into non-suppressive cells with a NK-like phenotype.

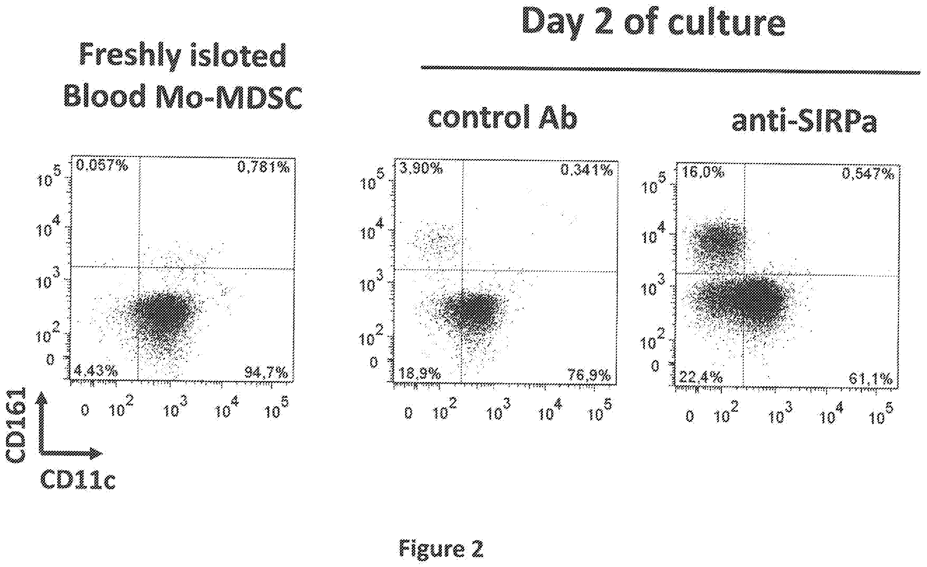

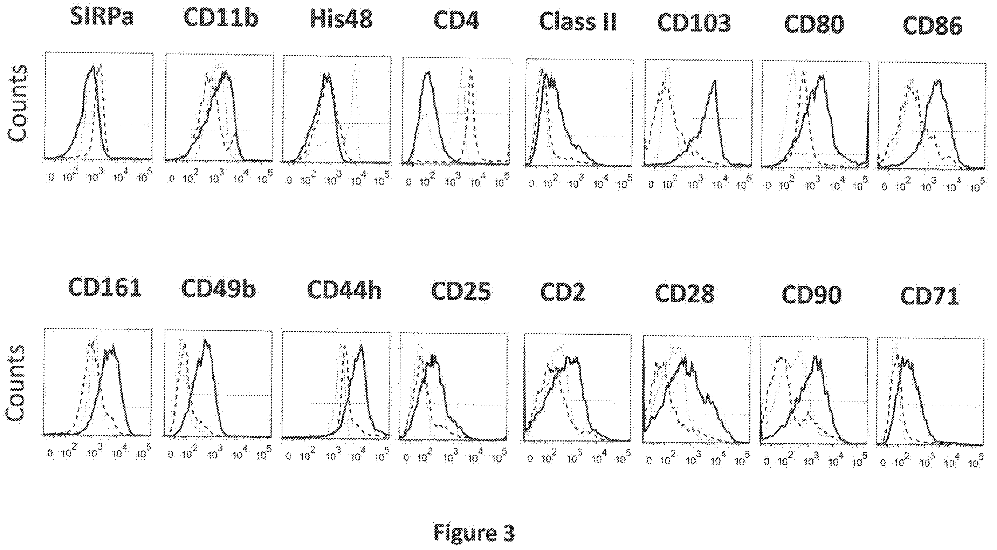

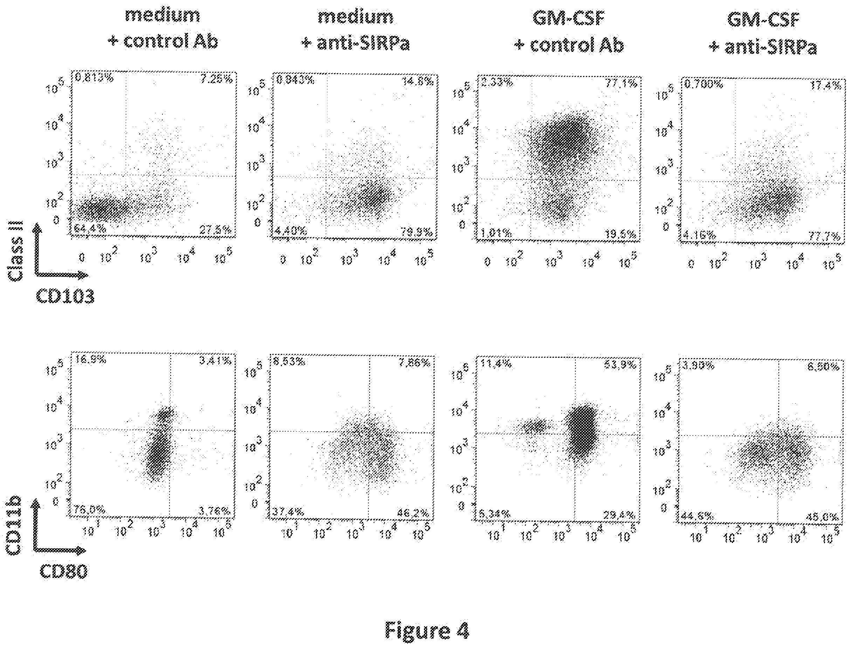

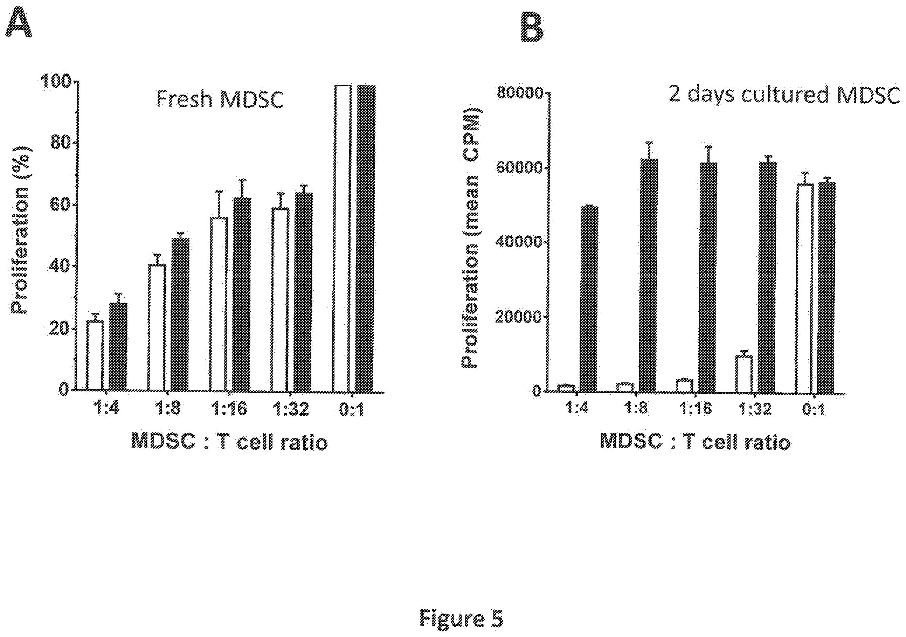

We first observed that monoclonal antibodies targeted at SIRPa and antagonizing its interaction with CD47 did not affect survival of Mo-MDSC in vitro, over a 48h period of time. In contrast, Mo-MDSC modified their phenotype in comparison to freshly isolated Mo-MDSC or to Mo-MDSC cultured over 48h with a control antibody (Figure 2 and 3). Whereas MDSC were previously described to differentiate into macrophages or tumor-associated macrophages (TAM), dendritic cells, or granulocytes, here we observed that anti-SIRPa mAbs differentiated Mo-MDSC did not display these phenotypes. In contrast incubation in the presence of GM-CSF did induce differentiation into the previously described phenotype. (Figure 4). Strikingly, in the rat species, anti-SIRPa-treated MDSC did not acquire high level of MHC Class II molecule, nor of CD68, and instead lost acquisition of CD4 marker. In humans, these cells lost CD11c expression and did not acquire HLA-DR. Unexpectedly, we observed these cells expressed high level of NK-specific markers, in particular CD161 and CD49b, and markers of mature cells (CD44h, CD103, CD80, CD86). They also expressed other marker of the lymphoid lineage (CD25, CD28, CD2) confirming these cells have been differentiated by anti-SIRPa monoclonal antibody into (non-myeloid) mature lymphoid cells. Furthermore, to confirm that anti-SIRPa induced differentiation in non-myeloid cells, we cultured MDSC with both GM-CSF and anti-SIRPa monoclonal antibodies. While GM-CSF alone induce the differentiation of MDSC in mature MHC Class II⁺ myeloids cells (i.d. dendritic cells and macrophages), we observed GM-CSF had no effect when associated with anti-SIRPa antibody and did not prevent the novel differentiation road induced by anti-SIRPa antibody (Figure 4). These results demonstrated that anti-SIRPa monoclonal antibody induce MDSC differentiation into cells with an effector NK-like (CD161⁺) phenotype and that this overcomes their conventional differentiation pathway. Finally, to confirm the mechanism of action of anti-SIRPa monoclonal antibodies, we first evaluated their ability to break immunosuppressive function of MDSC. While anti-SIRPa monoclonal antibodies did not modify the suppressive function of freshly isolated MDSC, we observed that MDSC differentiated in vitro by anti-SIRPa antibodies had lost their ability to suppress T-lymphocytes proliferation (Figure 5). To summarize, anti-SIRPa monoclonal antibodies induced the differentiation of Mo-MDSC into non-suppressive effector NK-like lymphoid cells.

### Anti-SIRPa monoclonal antibodies break MDSC-dependent immune tolerance in vivo

We previously described that kidney allograft tolerance induced by anti-CD28 monoclonal treatment in rat is maintained by the accumulation of Mo-MDSC (Dilek et al., 2012; Dugast et al., 2008). In order to confirm in vivo that anti-SIRPa monoclonal antibody could break MDSC-sustained immunodepression, independently of its effect on tumor elimination by improving macrophages phagocytosis, we treated tolerant kidney allograft recipient with anti-SIRPa monoclonal antibody or irrelevant control antibody. We observed that tolerant recipients rejected their allograft within two to three months after anti-SIRPa treatment, while graft function remained stable with control antibody (Figure 6). Peripheral blood immunophenotyping analysis of these animals confirmed our in-vitro observation, since we observed a significant decrease of MDSC after an average ten days of treatment with anti-SIRPa antibody treatment. Similarly, while NK cells did not express SIRPa, we observed a significant increase of NK lineage (CD161⁺) cells after an average ten days of treatment with anti-SIRPa antibody treatment. Histological examination of explanted grafts did not reveal expected acute cellular rejection mediated by T-lymphocytes. In contrast, we observed that T-lymphocyte infiltration originally present in the graft of tolerant recipients, was even less pronounced in the graft of rejected animals after anti-SIRPa antibody treatment (Figure 8). We previously described that peripheral MDSC accumulation in this model is associated with an accumulation of graft regulatory T cells (Dilek et al., 2012). Here we described that anti-SIRPa antibody treatment also indirectly modulates regulatory T cells, since these cells were barely detectable in the graft of anti-SIRPa treated recipient (Figure 9). Furthermore, while myeloid (CD11b/c) cells infiltration remains similar between groups, mature myeloid cells, such as macrophages, were more abundant in the graft of anti-SIRPa treated recipients. More importantly, while NK (CD161⁺) cells were barely detectable in the graft of tolerated recipients, we observed a significant graft infiltration in anti-SIRPa treated recipient, confirming in vitro studies and peripheral observation in vivo that anti-SIRPa antibody modulate both MDSC and NK cells.

### Anti-SIRPa monoclonal antibody treatment modulated tumor-infiltrating MDSC and NK cells and prevented mortality in cancer models

We administrated anti-SIRPa monoclonal antibody in murine models of hepatocellular carcinoma and melanoma. The hepatocellular carcinoma mouse model (Hepa1.6) is an aggressive cancer model inducing dead within two weeks after tumor cell line inoculation in the liver. In this model approved chemotherapeutic standard of care (e.g., Sorafenib) rescued an average of 60% of mice (Figure 10). In this stringent model, we observed that anti-SIRPa monoclonal antibody treatment in monotherapy significantly protected mice with an efficacy similar to Sorafenib. Interestingly, while tumor infiltration was significantly enhanced (in particular T-lymphocytes) after two weeks of treatment with anti-SIRPa antibody, we observed a significant decrease of MDSC within myeloid cells (Figure 11C). Furthermore, we observed also a significant increase of NK (CD161⁺) cells infiltration in these tumors as compared to control mice (Figure 12), in particular an accumulation of mature NK cells (CD11b⁺ CD27⁻) (Figure 13). In fact, it was previously described that the CD27⁺ CD11b⁻ phenotype corresponds to immature NK cells incapable of cytotoxicity and producing low level of cytokines, the CD11b⁺ CD27⁺ phenotype to effector NK cells producing cytokines but poorly cytotoxic, and the CD11b⁺ CD27⁻ phenotype to mature and highly cytotoxic NK cells (Desbois et al., 2012). To confirm these results in another tumor model, we used a B16 melanoma mouse model. Similarly, leukocytes extracted from the tumor of mice treated during two weeks with anti-SIRPa monoclonal antibody also showed intratumoral decrease of Mo-MDSC (Figure 15 B) and accumulation of NK (CD161⁺) cells (from 4,23% of the cells into the irrelevant antibody condition to 12,4% into anti-Sirpa treated animals (Figure 14)). Figure 15 A represents the overall survival rate of animals inoculated with melanoma and treated with an anti-PD-L1 or with an anti-SIRPa or with both during 4 weeks. Compared to the treatment with single molecules, the antibody combination showed a synergistic effect.

### Effect of the SIRPa blockade in an in vivo model of hepatocarcinoma

Figure 17A represents the overall survival rate of animals inoculated with hepatocarcinoma and treated with an anti-CD137, an anti-Sirpa or both during 4 weeks. 30% of anti-Sirpa treated animals survived more than 20 days after inoculation. This result is comparable to the results obtain when animals received the anti-CD137 antibody. Interestingly, 100% of the animals receiving the combo anti-Sirp+anti-CD137 survived. Compared to the results obtained with each molecule alone, this shows a strong synergistic effect of the 2 molecules.

Figure 17B represents the overall survival rate of animals inoculated with hepatocarcinoma and treated with an anti-PD-L1, an anti-Sirpa or both during 4 weeks. As observed before, 20% of anti-Sirpa treated animals survived more than 20 days after inoculation. The results showed a very interesting surviving rate when animals were treated with both molecules, compared to each single treatment. This result shows a synergistic effect of the anti-SIRPa antibody with the anti-PD-L1 antibody in a cancer model.

The in vivo experiments on 2 different cancer models showed that SIRPa is an interesting target for cancer treatment as monotherapy and even more when combined with other immunotherapies or chemotherapy. These results demonstrate that SIRPa is a new checkpoint which is important to block to the aim of inducing non suppressive cells into the tumor.

### REFERENCES

Adams S, Van der Laan LJ, Vernon-Wilson E, Renardel de Lavalette C, Dopp EA, et al. 1998. Signalregulatory protein is selectively expressed by myeloid and neuronal cells. J. Immunol. 161:1853-59
Apetoh, L., Végran, F., Ladoire, S., and Ghiringhelli, F. (2011). Restoration of antitumor immunity through selective inhibition of myeloid derived suppressor cells by anticancer therapies. Curr. Mol. Med. 11, 365-372.
Baniyash, M., Sade-Feldman, M., and Kanterman, J. (2014). Chronic inflammation and cancer: suppressing the suppressors. Cancer Immunol. Immunother. 63, 11-20.
Barclay, A.N., and Van den Berg, T.K. (2014). The interaction between signal regulatory protein alpha (SIRPa) and CD47: structure, function, and therapeutic target. Annu. Rev. Immunol. 32, 25-50.
Cai, W., Qin, A., Guo, P., Yan, D., Hu, F., Yang, Q., Xu, M., Fu, Y., Zhou, J., and Tang, X. (2013). Clinical significance and functional studies of myeloid-derived suppressor cells in chronic hepatitis C patients. J. Clin. Immunol. 33, 798-808.
Chao, M.P., Tang, C., Pachynski, R.K., Chin, R., Majeti, R., and Weissman, I.L. (2011). Extranodal dissemination of non-Hodgkin lymphoma requires CD47 and is inhibited by anti-CD47 antibody therapy. Blood 118, 4890-4901.
Chen, J., Deng, C., Shi, Q., Jiang, J., Zhang, Y., Shan, W., and Sun, W. (2013). CpG oligodeoxynucleotide induces bone marrow precursor cells into myeloid-derived suppressor cells. Mol Med Rep 8, 1149-1154.
Cheron, A., Floccard, B., Allaouchiche, B., Guignant, C., Poitevin, F., Malcus, C., Crozon, J., Faure, A., Guillaume, C., Marcotte, G., et al. (2010). Lack of recovery in monocyte human leukocyte antigen-DR expression is independently associated with the development of sepsis after major trauma. Crit Care 14, R208.
Cripps, J.G., and Gorham, J.D. (2011). MDSC in autoimmunity. Int. Immunopharmacol. 11, 789-793.
Cuenca, A.G., Delano, M.J., Kelly-Scumpia, K.M., Moreno, C., Scumpia, P.O., Laface, D.M., Heyworth, P.G., Efron, P.A., and Moldawer, L.L. (2011). A paradoxical role for myeloid-derived suppressor cells in sepsis and trauma. Mol. Med. 17, 281-292.
Diaz-Montero, C.M., Salem, M.L., Nishimura, M.I., Garrett-Mayer, E., Cole, D.J., and Montero, A.J. (2009). Increased circulating myeloid-derived suppressor cells correlate with clinical cancer stage, metastatic tumor burden, and doxorubicin-cyclophosphamide chemotherapy. Cancer Immunol. Immunother. 58, 49-59.
Diaz-Montero, C.M., Finke, J., and Montero, A.J. (2014). Myeloid-derived suppressor cells in cancer: therapeutic, predictive, and prognostic implications. Semin. Oncol. 41, 174-184.
Dilek, N., van Rompaey, N., Le Moine, A., and Vanhove, B. (2010). Myeloid-derived suppressor cells in transplantation. Curr Opin Organ Transplant 15, 765-768.
Dugast, A.-S., Haudebourg, T., Coulon, F., Heslan, M., Haspot, F., Poirier, N., Vuillefroy de Silly, R., Usal, C., Smit, H., Martinet, B., et al. (2008). Myeloid-derived suppressor cells accumulate in kidney allograft tolerance and specifically suppress effector T cell expansion. J. Immunol 180, 7898-7906.
Edris, B., Weiskopf, K., Volkmer, A.K., Volkmer, J.-P., Willingham, S.B., Contreras-Trujillo, H., Liu, J., Majeti, R., West, R.B., Fletcher, J.A., et al. (2012). Antibody therapy targeting the CD47 protein is effective in a model of aggressive metastatic leiomyosarcoma. Proc. Natl. Acad. Sci. U.S.A. 109, 6656-6661.
Gabitass, R.F., Annels, N.E., Stocken, D.D., Pandha, H.A., and Middleton, G.W. (2011). Elevated myeloid-derived suppressor cells in pancreatic, esophageal and gastric cancer are an independent prognostic factor and are associated with significant elevation of the Th2 cytokine interleukin-13. Cancer Immunol. Immunother. 60, 1419-1430.
Gabrilovich, D.I., and Nagaraj, S. (2009). Myeloid-derived suppressor cells as regulators of the immune system. Nat. Rev. Immunol. 9, 162-174.
Gabrilovich, D.I., Velders, M.P., Sotomayor, E.M., and Kast, W.M. (2001). Mechanism of immune dysfunction in cancer mediated by immature Gr-1+ myeloid cells. J. Immunol. 166, 5398-5406.
Gabrilovich, D.I., Bronte, V., Chen, S.-H., Colombo, M.P., Ochoa, A., Ostrand-Rosenberg, S., and Schreiber, H. (2007). The terminology issue for myeloid-derived suppressor cells. Cancer Res. 67, 425; author reply 426.
Gabrilovich, D.I., Ostrand-Rosenberg, S., and Bronte, V. (2012). Coordinated regulation of myeloid cells by tumours. Nat. Rev. Immunol. 12, 253-268.
Garg, A., and Spector, S.A. (2014). HIV type 1 gp120-induced expansion of myeloid derived suppressor cells is dependent on interleukin 6 and suppresses immunity. J. Infect. Dis. 209, 441-451.
Van Ginderachter, J.A., Beschin, A., De Baetselier, P., and Raes, G. (2010). Myeloid-derived suppressor cells in parasitic infections. Eur. J. Immunol. 40, 2976-2985.
Goh, C., Narayanan, S., and Hahn, Y.S. (2013). Myeloid-derived suppressor cells: the dark knight or the joker in viral infections? Immunol. Rev. 255, 210-221.
Heithoff, D.M., Enioutina, E.Y., Bareyan, D., Daynes, R.A., and Mahan, M.J. (2008). Conditions that diminish myeloid-derived suppressor cell activities stimulate cross-protective immunity. Infect. Immun. 76, 5191-5199.
Highfill, S.L., Cui, Y., Giles, A.J., Smith, J.P., Zhang, H., Morse, E., Kaplan, R.N., and Mackall, C.L. (2014). Disruption of CXCR2-mediated MDSC tumor trafficking enhances anti-PD1 efficacy. Sci Transl Med 6, 237ra67.
Hock BD, McKenzie JL, Cross NB, Currie MJDynamic changes in myeloid derived suppressor cell subsets following renal transplant: A prospective study. Transpl Immunol. 2015 Jun;32(3):164-71
Hu, X., Bardhan, K., Paschall, A.V., Yang, D., Waller, J.L., Park, M.A., Nayak-Kapoor, A., Samuel, T.A., Abrams, S.I., and Liu, K. (2013). Deregulation of Apoptotic Factors Bcl-xL and Bax Confers Apoptotic Resistance to Myeloid-derived Suppressor Cells and Contributes to Their Persistence in Cancer. J. Biol. Chem. 288, 19103-19115.
Huang, A., Zhang, B., Wang, B., Zhang, F., Fan, K.-X., and Guo, Y.-J. (2013). Increased CD14(+)HLA-DR (-/low) myeloid-derived suppressor cells correlate with extrathoracic metastasis and poor response to chemotherapy in non-small cell lung cancer patients. Cancer Immunol. Immunother. 62, 1439-1451.
Idorn, M., Køllgaard, T., Kongsted, P., Sengeløv, L., and Thor Straten, P. (2014). Correlation between frequencies of blood monocytic myeloid-derived suppressor cells, regulatory T cells and negative prognostic markers in patients with castration-resistant metastatic prostate cancer. Cancer Immunol. Immunother.
James, B.R., Anderson, K.G., Brincks, E.L., Kucaba, T.A., Norian, L.A., Masopust, D., and Griffith, T.S. (2014). CpG-mediated modulation of MDSC contributes to the efficacy of Ad5-TRAIL therapy against renal cell carcinoma. Cancer Immunol. Immunother.
Janols, H., Bergenfelz, C., Allaoui, R., Larsson, A.-M., Rydén, L., Björnsson, S., Janciauskiene, S., Wullt, M., Bredberg, A., and Leandersson, K. (2014). A high frequency of MDSCs in sepsis patients, with the granulocytic subtype dominating in gram-positive cases. J. Leukoc. Biol.
Janssen WJ, McPhillips KA, DickinsonMG,Linderman DJ, Morimoto K, et al. 2008. Surfactant proteins A andDsuppress alveolar macrophage phagocytosis via interaction with SIRPa.Am. J. Respir. Crit. Care Med. 178:158-67
Kalathil, S., Lugade, A.A., Miller, A., Iyer, R., and Thanavala, Y. (2013). Higher frequencies of GARP(+)CTLA-4(+)Foxp3(+) T regulatory cells and myeloid-derived suppressor cells in hepatocellular carcinoma patients are associated with impaired T-cell functionality. Cancer Res. 73, 2435-2444.
Keskinov, A.A., and Shurin, M.R. (2014). Myeloid regulatory cells in tumor spreading and metastasis. Immunobiology.
Khaled, Y.S., Ammori, B.J., and Elkord, E. (2013). Myeloid-derived suppressor cells in cancer: recent progress and prospects. Immunol. Cell Biol. 91, 493-502.
Kitano, S., Postow, M.A., Ziegler, C.G.K., Kuk, D., Panageas, K.S., Cortez, C., Rasalan, T., Adamow, M., Yuan, J., Wong, P., et al. (2014). Computational algorithm-driven evaluation of monocytic myeloid-derived suppressor cell frequency for prediction of clinical outcomes. Cancer Immunol Res 2, 812-821.
Kurkó, J., Vida, A., Glant, T.T., Scanzello, C.R., Katz, R.S., Nair, A., Mikecz, K., and Szekanecz, Z. (2014). Identification of myeloid-derived suppressor cells in the synovial fluid of patients with rheumatoid arthritis: a pilot study. BMC Musculoskelet Disord 15, 281.
Kurmaeva, E., Bhattacharya, D., Goodman, W., Omenetti, S., Merendino, A., Berney, S., Pizarro, T., and Ostanin, D.V. (2014). Immunosuppressive monocytes: possible homeostatic mechanism to restrain chronic intestinal inflammation. J. Leukoc. Biol. 96, 377-389.
Majeti, R., Chao, M.P., Alizadeh, A.A., Pang, W.W., Jaiswal, S., Gibbs, K.D., Jr, van Rooijen, N., and Weissman, I.L. (2009). CD47 is an adverse prognostic factor and therapeutic antibody target on human acute myeloid leukemia stem cells. Cell 138, 286-299.
Makarenkova, V.P., Bansal, V., Matta, B.M., Perez, L.A., and Ochoa, J.B. (2006). CD11b+/Gr-1+ myeloid suppressor cells cause T cell dysfunction after traumatic stress. J. Immunol. 176, 2085-2094.
Mao, Y., Sarhan, D., Steven, A., Seliger, B., Kiessling, R., and Lundqvist, A. (2014). Inhibition of tumor-derived prostaglandin-e2 blocks the induction of myeloid-derived suppressor cells and recovers natural killer cell activity. Clin. Cancer Res. 20, 4096-4106.
Milling S, Yrlid U, Cerovic V, MacPherson G. 2010. Subsets of migrating intestinal dendritic cells. Immunol. Rev. 234:259-67
Mirza, N., Fishman, M., Fricke, I., Dunn, M., Neuger, A.M., Frost, T.J., Lush, R.M., Antonia, S., and Gabrilovich, D.I. (2006). All-trans-retinoic acid improves differentiation of myeloid cells and immune response in cancer patients. Cancer Res. 66, 9299-9307.
Nefedova, Y., Fishman, M., Sherman, S., Wang, X., Beg, A.A., and Gabrilovich, D.I. (2007). Mechanism of all-trans retinoic acid effect on tumor-associated myeloid-derived suppressor cells. Cancer Res. 67, 11021-11028.
Ochando, J.C., and Chen, S.H. (2012). Myeloid-derived suppressor cells in transplantation and cancer. Immunol. Res. 54, 275-285.
Oldenborg, P.A., Zheleznyak, A., Fang, Y.F., Lagenaur, C.F., Gresham, H.D., and Lindberg, F.P. (2000). Role of CD47 as a marker of self on red blood cells. Science 288, 2051-2054.
Ostrand-Rosenberg, S., Sinha, P., Chornoguz, O., and Ecker, C. (2012). Regulating the suppressors: apoptosis and inflammation govern the survival of tumor-induced myeloid-derived suppressor cells (MDSC). Cancer Immunol. Immunother. 61, 1319-1325.
Seiffert M, Cant C, Chen Z, Rappold I, Brugger W, et al. 1999. Human signalregulatory protein is expressed on normal, but not on subsets of leukemic myeloid cells and mediates cellular adhesion involving its counterreceptor CD47. Blood 94:3633-43
Serafini, P. (2013). Myeloid derived suppressor cells in physiological and pathological conditions: the good, the bad, and the ugly. Immunol. Res. 57, 172-184.
Shen, P., Wang, A., He, M., Wang, Q., and Zheng, S. (2014). Increased circulating Lin(-/low) CD33(+) HLA-DR(-) myeloid-derived suppressor cells in hepatocellular carcinoma patients. Hepatol. Res. 44, 639-650.
Smith, A.R., and Reynolds, J.M. (2014). Editorial: The contribution of myeloid-derived suppression to inflammatory disease. J. Leukoc. Biol. 96, 361-364.
Solito, S., Marigo, I., Pinton, L., Damuzzo, V., Mandruzzato, S., and Bronte, V. (2014). Myeloid-derived suppressor cell heterogeneity in human cancers. Ann. N. Y. Acad. Sci. 1319, 47-65.
Srivastava, M.K., Zhu, L., Harris-White, M., Kar, U.K., Kar, U., Huang, M., Johnson, M.F., Lee, J.M., Elashoff, D., Strieter, R., et al. (2012a). Myeloid suppressor cell depletion augments antitumor activity in lung cancer. PLoS ONE 7, e40677.
Srivastava, M.K., Dubinett, S., and Sharma, S. (2012b). Targeting MDSCs enhance therapeutic vaccination responses against lung cancer. Oncoimmunology 1, 1650-1651.
Sui, Y., Hogg, A., Wang, Y., Frey, B., Yu, H., Xia, Z., Venzon, D., McKinnon, K., Smedley, J., Gathuka, M., et al. (2014). Vaccine-induced myeloid cell population dampens protective immunity to SIV. J. Clin. Invest. 124, 2538-2549.
Sun, H.-L., Zhou, X., Xue, Y.-F., Wang, K., Shen, Y.-F., Mao, J.-J., Guo, H.-F., and Miao, Z.-N. (2012). Increased frequency and clinical significance of myeloid-derived suppressor cells in human colorectal carcinoma. World J. Gastroenterol. 18, 3303-3309.
Talmadge, J.E., and Gabrilovich, D.I. (2013). History of myeloid-derived suppressor cells. Nat. Rev. Cancer 13, 739-752.
Taylor, J.V., Gordon, L.E., and Polk, H.C. (2000). Early decrease in surface expression of HLA-DQ predicts the development of infection in trauma patients. Clin. Exp. Immunol. 122, 308-311.
Tseng, D., Volkmer, J.-P., Willingham, S.B., Contreras-Trujillo, H., Fathman, J.W., Fernhoff, N.B., Seita, J., Inlay, M.A., Weiskopf, K., Miyanishi, M., et al. (2013). Anti-CD47 antibody-mediated phagocytosis of cancer by macrophages primes an effective antitumor T-cell response. Proc. Natl. Acad. Sci. U.S.A. 110, 11103-11108.
Uluçkan, O., Becker, S.N., Deng, H., Zou, W., Prior, J.L., Piwnica-Worms, D., Frazier, W.A., and Weilbaecher, K.N. (2009). CD47 regulates bone mass and tumor metastasis to bone. Cancer Res. 69, 3196-3204.
Venet, F., Tissot, S., Debard, A.-L., Faudot, C., Crampé, C., Pachot, A., Ayala, A., and Monneret, G. (2007). Decreased monocyte human leukocyte antigen-DR expression after severe burn injury: Correlation with severity and secondary septic shock. Crit. Care Med. 35, 1910-1917.
Vincent, J., Mignot, G., Chalmin, F., Ladoire, S., Bruchard, M., Chevriaux, A., Martin, F., Apetoh, L., Rébé, C., and Ghiringhelli, F. (2010). 5-Fluorouracil selectively kills tumor-associated myeloid-derived suppressor cells resulting in enhanced T cell-dependent antitumor immunity. Cancer Res. 70, 3052-3061.
Vollbrecht, T., Stirner, R., Tufman, A., Roider, J., Huber, R.M., Bogner, J.R., Lechner, A., Bourquin, C., and Draenert, R. (2012). Chronic progressive HIV-1 infection is associated with elevated levels of myeloid-derived suppressor cells. AIDS 26, F31-37.
Wang, Y., Xu, Z., Guo, S., Zhang, L., Sharma, A., Robertson, G.P., and Huang, L. (2013). Intravenous delivery of siRNA targeting CD47 effectively inhibits melanoma tumor growth and lung metastasis. Mol. Ther. 21, 1919-1929.
Weide, B., Martens, A., Zelba, H., Stutz, C., Derhovanessian, E., Di Giacomo, A.M., Maio, M., Sucker, A., Schilling, B., Schadendorf, D., et al. (2014). Myeloid-derived suppressor cells predict survival of patients with advanced melanoma: comparison with regulatory T cells and NY-ESO-1- or melan-A-specific T cells. Clin. Cancer Res. 20, 1601-1609.
Weiskopf, K., Ring, A.M., Ho, C.C.M., Volkmer, J.-P., Levin, A.M., Volkmer, A.K., Ozkan, E., Fernhoff, N.B., van de Rijn, M., Weissman, I.L., et al. (2013). Engineered SIRPa variants as immunotherapeutic adjuvants to anticancer antibodies. Science 341, 88-91.
Whitfield-Larry, F., Felton, J., Buse, J., and Su, M.A. (2014). Myeloid-derived suppressor cells are increased in frequency but not maximally suppressive in peripheral blood of Type 1 Diabetes Mellitus patients. Clin. Immunol. 153, 156-164.
Willingham, S.B., Volkmer, J.-P., Gentles, A.J., Sahoo, D., Dalerba, P., Mitra, S.S., Wang, J., Contreras-Trujillo, H., Martin, R., Cohen, J.D., et al. (2012). The CD47-signal regulatory protein alpha (SIRPa) interaction is a therapeutic target for human solid tumors. Proc. Natl. Acad. Sci. U.S.A. 109, 6662-6667.
Ye, X.-Z., Yu, S.-C., and Bian, X.-W. (2010). Contribution of myeloid-derived suppressor cells to tumor-induced immune suppression, angiogenesis, invasion and metastasis. J Genet Genomics 37, 423-430.
Youn, J.-I., Kumar, V., Collazo, M., Nefedova, Y., Condamine, T., Cheng, P., Villagra, A., Antonia, S., McCaffrey, J.C., Fishman, M., et al. (2013). Epigenetic silencing of retinoblastoma gene regulates pathologic differentiation of myeloid cells in cancer. Nat. Immunol. 14, 211-220.
Zhang, B., Wang, Z., Wu, L., Zhang, M., Li, W., Ding, J., Zhu, J., Wei, H., and Zhao, K. (2013). Circulating and tumor-infiltrating myeloid-derived suppressor cells in patients with colorectal carcinoma. PLoS ONE 8, e57114.
Zhao, X.W., van Beek, E.M., Schornagel, K., Van der Maaden, H., Van Houdt, M., Otten, M.A., Finetti, P., Van Egmond, M., Matozaki, T., Kraal, G., et al. (2011). CD47-signal regulatory protein-α (SIRPa) interactions form a barrier for antibody-mediated tumor cell destruction. Proc. Natl. Acad. Sci. U.S.A. 108, 18342-18347.
Zhu, X., Pribis, J.P., Rodriguez, P.C., Morris, S.M., Vodovotz, Y., Billiar, T.R., and Ochoa, J.B. (2014). The central role of arginine catabolism in T-cell dysfunction and increased susceptibility to infection after physical injury. Ann. Surg. 259, 171-178.

## Claims

1. A compound selected from the group consisting of an antibody and an antigen-binding fragment thereof, wherein the compound:
- specifically binds to the signal regulatory protein alpha (SIRPa), and
- blocks the interaction between SIRPa and CD47,
for use in the treatment of solid cancer in a patient, the solid cancer being selected from the group consisting of carcinoma, adenocarcinoma, breast cancer, prostate cancer, ovarian cancer, lung cancer, pancreatic cancer, colon cancer, sarcoma, lymphoma and blastoma.

2. The compound of claim 1 for the use according to claim 1, wherein the compound induces the differentiation of MDSCs into cells expressing at least one human marker selected from the group consisting of CD80, CD86 and CD103.

3. The compound of claim 1 for the use according to claim 1, wherein the compound induces the differentiation of MDSCs into cells expressing the CD80, CD86 and CD103 human markers.

4. The compound of claim 2 or 3 for the use according to claim 2 or 3, wherein the compound induces the differentiation of MDSCs into cells expressing the human marker CD11b.

5. The compound of any one of claims 1 to 4 for the use according to any one of claims 1 to 4, wherein the cancer is metastatic.

6. The compound of any one of claims 1 to 5 for the use according to any one of claims 1 to 5, wherein said compound is combined with a second therapeutic agent.

7. The compound of claim 6, for the use according to claim 6, wherein said second therapeutic agent is selected from the group consisting of chemotherapeutic agents, radiotherapy, surgery, immunotherapeutic agents, antibiotics and probiotics.

## Patentansprüche

1. Verbindung, ausgewählt aus der Gruppe bestehend aus einem Antikörper und einem Antigen-bindenden Fragment davon, wobei die Verbindung:
- spezifisch an das Signalregulationsprotein Alpha (SIRPa) bindet und
- die Interaktion zwischen SIRPa und CD47 blockiert,
zur Verwendung bei der Behandlung von soliden Tumoren bei einem Patienten, wobei der solide Tumor ausgewählt ist aus der Gruppe bestehend aus Karzinom, Adenokarzinom, Brustkrebs, Prostatakrebs, Eierstockkrebs, Lungenkrebs, Bauchspeicheldrüsenkrebs, Darmkrebs, Sarkom, Lymphom und Blastom.

2. Verbindung nach Anspruch 1 zur Verwendung gemäß Anspruch 1, wobei die Verbindung die Differenzierung von MDSCs zu Zellen induziert, welche mindestens einen humanen Marker exprimieren, ausgewählt aus der Gruppe bestehend aus CD80, CD86 und CD103.

3. Verbindung nach Anspruch 1 zur Verwendung gemäß Anspruch 1, wobei die Verbindung die Differenzierung von MDSCs zu Zellen induziert, welche die humanen Marker CD80, CD86 und CD103 exprimieren.

4. Verbindung nach Anspruch 2 oder 3 zur Verwendung gemäß Anspruch 2 oder 3, wobei die Verbindung die Differenzierung von MDSCs zu Zellen induziert, welche den humanen Marker CD11b exprimieren.

5. Verbindung nach einem der Ansprüche 1 bis 4 zur Verwendung gemäß einem der Ansprüche 1 bis 4, wobei der Krebs metastasierend ist.

6. Verbindung nach einem der Ansprüche 1 bis 5 zur Verwendung gemäß einem der Ansprüche 1 bis 5, wobei die Verbindung mit einem zweiten therapeutischen Mittel kombiniert wird.

7. Verbindung nach Anspruch 6 zur Verwendung gemäß Anspruch 6, wobei das zweite therapeutische Mittel ausgewählt ist aus der Gruppe bestehend aus chemotherapeutischen Mitteln, Strahlentherapie, Chirurgie, immuntherapeutischen Mitteln, Antibiotika und Probiotika.

## Revendications

1. Composé choisi dans le groupe consistant en un anticorps et un fragment de liaison à l'antigène de celui-ci, dans lequel le composé :
- se lie spécifiquement à la protéine régulatrice de signal alpha (SIRPa), et
- bloque l'interaction entre SIRPa et CD47,
pour une utilisation dans le traitement d'un cancer solide chez un patient, le cancer solide étant choisi dans le groupe consistant en carcinome, adénocarcinome, cancer du sein, cancer de la prostate, cancer de l'ovaire, cancer du poumon, cancer du pancréas, cancer du côlon, sarcome, lymphome et blastome.

2. Composé selon la revendication 1 pour une utilisation selon la revendication 1, dans lequel le composé induit la différenciation de MDSC en cellules exprimant au moins un marqueur humain choisi dans le groupe consistant en CD80, CD86 et CD103.

3. Composé selon la revendication 1 pour l'utilisation selon la revendication 1, dans lequel le composé induit la différenciation de MDSC en cellules exprimant les marqueurs humains CD80, CD86 et CD103.

4. Composé selon la revendication 2 ou 3 pour l'utilisation selon la revendication 2 ou 3, dans lequel le composé induit la différenciation de MDSC en cellules exprimant le marqueur humain CD11b.

5. Composé selon l'une quelconque des revendications 1 à 4 pour l'utilisation selon l'une quelconque des revendications 1 à 4, dans lequel le cancer est métastatique.

6. Composé selon l'une quelconque des revendications 1 à 5 pour l'utilisation selon l'une quelconque des revendications 1 à 5, dans lequel ledit composé est combiné avec un deuxième agent thérapeutique.

7. Composé selon la revendication 6, pour l'utilisation selon la revendication 6, dans lequel ledit deuxième agent thérapeutique est choisi dans le groupe consistant en les agents chimiothérapeutiques, la radiothérapie, la chirurgie, les agents immunothérapeutiques, les antibiotiques et les probiotiques.
